# EUROPÄISCHE PATENTSCHRIFT

(11) **EP 0 892 786 B1**
(45) Veröffentlichungstag und Bekanntmachung des Hinweises auf die Patenterteilung: **09.06.2004**
(21) Anmeldenummer: 97915481.2
(22) Anmeldetag: 04.04.1997
(51) Int. Cl.: C07D 239/52, C07D 239/34, C07D 239/60, C07D 239/70, C07D 403/12, A61K 31/505

(54) **NEUE CARBONSÄUREDERIVATE, IHRE HERSTELLUNG UND VERWENDUNG**
NEW CARBOXYLIC ACID DERIVATIVES, THEIR PRODUCTION AND USE
NOUVEAUX DERIVES D'ACIDES CARBOXYLIQUES, LEUR PREPARATION ET LEUR UTILISATION

(30) Priorität: 12.04.1996 DE 19614534
(43) Veröffentlichungstag der Anmeldung: 27.01.1999
(73) Patentinhaber: Abbott GmbH & Co. KG, 65205 Wiesbaden (DE)
(72) Erfinder: RIECHERS, Hartmut, D-67435 Neustadt (DE); KLINGE, Dagmar, D-69120 Heidelberg (DE); AMBERG, Wilhelm, D-61381 Friedrichsdorf (DE); KLING, Andreas, D-68239 Mannheim (DE); HILLEN, Heinz, D-67454 Ha loch (DE); UNGER, Liliane, D-67065 Ludwigshafen (DE); ELGER, Bernd, D-67433 Neustadt (DE)
(74) Vertreter: Grünecker, Kinkeldey, Stockmair & Schwanhäusser Anwaltssozietät
(86) Internationale Anmeldenummer: PCT/EP1997/001684
(87) Internationale Veröffentlichungsnummer: WO 1997/038980

(56) Entgegenhaltungen:
- EP-A- 0 481 512
- DE-A- 19 533 023
- JOURNAL OF MEDICINAL CHEMISTRY, Bd. 39, Nr. 11, Mai 1996, WASHINGTON US, Seiten 2123-2128, XP002034887 H.RIECHERS ET AL.: "DISCOVERY AND OPTIMIZATION OF A NOVEL CLASS"
- 'Roche Lexicon Medizin, 2. Auflage', 1984, URBAN UND SCHWARZENBERG
- 'Pschyrembel Klinisches Wörterbuch, 256. Auflage', 1990, DE GRUYTER

## Beschreibung

Die vorliegende Erfindung betrifft neue Carbonsäuredrivate, deren Herstellung und Verwendung.

Endothelin ist ein aus 21 Aminosäuren aufgebautes Peptid, das von vaskulärem Endothel synthetisiert und freigesetzt wird. Endothelin existiert in drei Isoformen, ET-1, ET-2 und ET-3. Im Folgenden bezeichnet "Endothelin" oder "ET" eine oder alle Isoformen von Endothelin. Endothelin ist ein potenter Vasokonstriktor und hat einen starken Effekt auf den Gefäßtonus. Es ist bekannt, daß diese Vasokonstriktion von der Bindung von Endothelin an seinen Rezeptor verursacht wird (Nature, 332, 411-415, 1988; FEBS Letters, 231, 440-444, 1988 und Biochem. Biophys. Res. Commun., 154, 868-875, 1988).

Erhöhte oder abnormale Freisetzung von Endothelin verursacht eine anhaltende Gefäßkontraktion in peripheren, renalen und zerebralen Blutgefäßen, die zu Krankheiten führen kann. Wie in der Literatur berichtet, ist Endothelin in eine Reihe von Krankheiten involviert; dazu zählen Hypertonie, Myokardinfarkt, Herzversagen, Nierenversagen, pulmonäre Hypertonie, Raynaud-Syndrom, zerebrale Vasospasmen, Atherosklerose, Schlaganfall, benigne Prostatahypertrophie und Asthma (Japan J. Hypertension 12, 79 (1989), J. Vascular Med. Biology 2, 207 (1990), J. Am. Med. Association 264, 2868 (1990), Nature 344, 11 (1990), N. Engl. J. Med. 322, 205 (1989), N. Engl. J. Med. 328, 1732 (1993), Nephron 66, 373 (1994), Strake 25, 904 (1994), Nature 365, 759 (1993), J. Mol. Cell. Cardiol. 27, A234 (1995), Cancer Research 56, 663 (1996).

Demnach sollten Substanzen, die spezifisch die Bindung von Endothelin an den Rezeptor inhibieren, auch die obengenannten verschiedenen physiologischen Effekte von Endothelin antagonisieren und daher wertvolle Pharmaka darstellen.

In der deutschen Patentanmeldung mit dem Aktenzeichen P 44 36 851.8 sind folgende Verbindungen als Endothelinrezeptorantagonisten beschrieben:

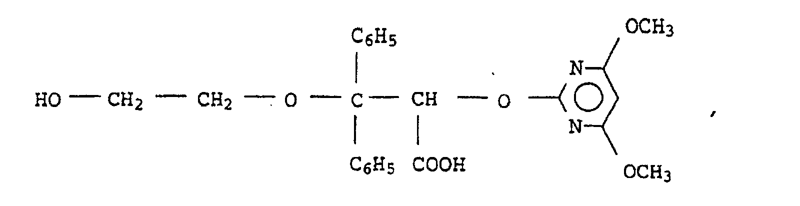

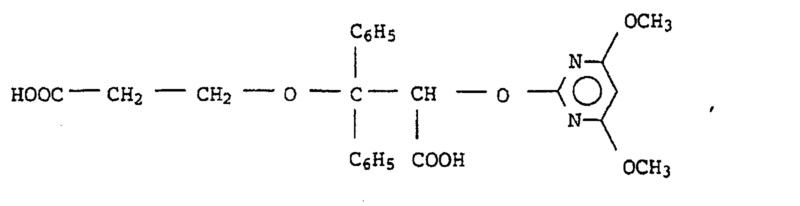

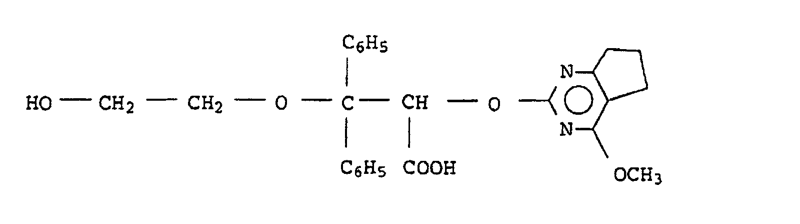

Es wurde nun gefunden, daß bestimmte Carbonsäurederivate gute Hemmstoffe für Endothelinrezeptoren sind und daß diese Verbindungen gleichzeitig eine relativ niedrige Plasmabindung aufweisen.

Gegenstand der Erfindung sind Carbonsäurederivate der Formel I 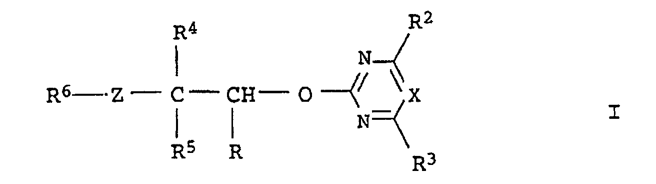 in der R eine Formylgruppe, Tetrazol, Nitril, eine Gruppe COOH oder einen zu COOH hydrolysierbaren Rest bedeutet und die übrigen Substituenten folgende Bedeutung haben:
- R²: Halogen, C₁-C₄-Alkyl, C₁-C₄-Halogenalkyl, C₁-C₄-Alkoxy, C₁-C₄-Halogenalkoxy oder C₁-C₄-Alkylthio;
- X: Stickstoff oder CR¹⁴, worin R¹⁴ Wasserstoff oder C₁₋₅-Alkyl bedeutet oder CR¹⁴ zusammen mit CR³ einen 5- oder 6-gliedrigen Alkylen- oder Alkenylenring bildet, der durch eine oder zwei C₁₋₄-Alkylgruppen substituiert sein kann und worin jeweils eine Methylengruppe durch Sauerstoff, Schwefel, -NH oder -NC₁₋₄-Alkyl ersetzt sein kann;
- R³: Halogen, C₁-C₄-Alkyl, C₁-C₄-Halogenalkyl, C₁-C₄-Alkoxy, C₁-C₄-Halogenalkoxy, -NH-O-C₁₋₄-Alkyl, C₁-C₄-Alkylthio oder CR³ ist mit CR¹⁴ wie oben angegeben zu einem 5- oder 6-gliedrigen Ring verknüpft;
- R⁴ und R⁵: (die gleich oder verschieden sein können):
Phenyl oder Naphthyl, die durch einen oder mehrere der folgenden Reste substituiert sein können: Halogen, Nitro, Cyano, Hydroxy, C₁-C₄-Alkyl, C₁-C₄-Halogenalkyl, C₁-C₄-Alkoxy, C₁-C₄-Halogenalkoxy, Phenoxy, C₁-C₄-Alkylthio, Amino, C₁-C₄-Alkylamino oder C₁-C₄-Dialkylamino; oder
Phenyl oder Naphthyl, die orthoständig über eine direkte Bindung, eine Methylen-, Ethylen- oder Ethenylengruppe, ein Sauerstoff- oder Schwefelatom oder eine SO₂-, NH- oder N-Alkyl-Gruppe miteinander verbunden sind, oder C₃-C₇-Cycloalkyl;
- R⁶: C₁-C₁₀-Alkyl, C₃-C₁₀-Alkenyl oder C₃-C₁₀-Alkinyl, wobei die Reste jeweils ein- oder mehrfach substituiert sind durch Hydroxy, Mercapto, Carboxy, 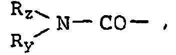 wobei R_{y} und R_{z} unabhängig voneinander Wasserstoff oder C₁-C₅-Alkyl bedeuten; Sulfonyl, Cyano, Guanidino, C₁-C₄-Alkoxy;
- Z: Schwefel oder Sauerstoff,
mit der Maßgabe, dass Carbonsäurederivate der Formel I ausgeschlossen sind, wenn der Rest R COOH, der Rest R² OMe, die Reste R⁴ und R⁵ Phenyl, und die Reste Y und Z O sind, und
(1) gleichzeitig der Rest R⁶ CH₂-CH₂-SO₂-CH(CH₃)₂, der Rest R³ OMe und der Rest X CH sind, oder
(2) gleichzeitig der Rest R⁶ CH₂-CH₂-SO₂-CH(CH₃)₂, der Rest R³ NH-OCH₃ und der Rest X CH sind, oder
(3) gleichzeitig der Rest R⁶ OH-CH₂-CH₂, der Rest R³ OMe und der Rest X CH sind, oder
(4) gleichzeitig der Rest R⁶ HOOC-(CH₂)₂-, der Rest R³ OMe und der Rest X CH sind, oder
(5) gleichzeitig der Rest R⁶ OH-CH₂-CH₂ und die Reste R³ und X zusammen einen Rest CH₂-CH₂-CH₂-C bilden sind.

Die Verbindungen und auch die Zwischenprodukte zu ihrer Herstellung, wie z.B. IV und VI, können ein oder mehrere asymmetrische substituierte Kohlenstoffatome besitzen. Solche Verbindungen können als reine Enantiomere bzw. reine Diastereomere oder als deren Mischung vorliegen. Bevorzugt ist die Verwendung einer enantiomerenreinen Verbindung als Wirkstoff.

Gegenstand der Erfindung ist weiter die Verwendung der oben genannten Carbonsäurederivate zur Herstellung von Arzneimitteln, insbesondere zur Herstellung von Hemmstoffen für Endothelinrezeptoren.

Die Herstellung der erfindungsgemäßen Verbindungen geht aus von den Epoxiden IV, die man in allgemein bekannter Weise, z.B. wie in J. March, Advanced Organic Chemistry, 2nd ed., 1983, S. 862 und S. 750 beschrieben, aus den Ketonen II oder den Olefinen III erhält:

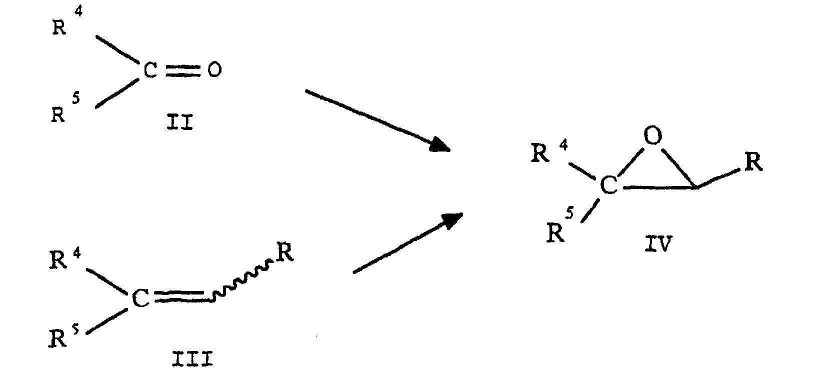

Carbonsäurederivate der allgemeinen Formel VI können hergestellt werden, indem man die Epoxide der allgemeinen Formel IV (z.B. mit R = ROOR¹⁰) mit Alkoholen oder Thiolen der allgemeinen Formel V, in der R⁶ und Z die in Anspruch 1 genannte Bedeutung haben, zur Reaktion bringt.

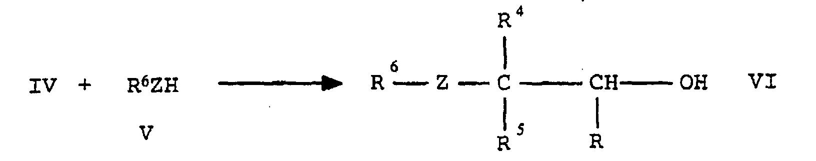

Dazu werden Verbindungen der allgemeinen Formel IV mit einer Verbindungen der Formel V, im Molverhältnis von etwa 1:1 bis 1:7, bevorzugt 1 bis 3 Moläquivalenten, auf eine Temperatur von 50 bis 200°C, bevorzugt 80 bis 150°C, erhitzt.

Weitere funktionelle Gruppen in R⁶ sind bei der Reaktion mit Verbindungen der Formel IV zunächst in allgemein bekannter Weise geschützt; beispielsweise können Alkohole als Acetate, Diole als Acetale und Carboxylgruppen als Ester geschützt werden. Die Schutzgruppen können nach der Reaktion von Verbindungen der Formel VI mit VII abgespalten werden.

Die Reaktion kann auch in Gegenwart eines Verdünnungsmittels erfolgen. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylchlorid und Trichlorethylen, Ether, wie zum Beispiel Diisopropylether, Dibutylether, Methyl-tert.-Butylether-Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfoxide und Sulfone, wie zum Beispiel Dimethylsulfoxid und Sulfolan, Basen, wie zum Beispiel Pyridin, N-Methylpyrrolidon, cyclische Harnstoffe wie 1,3-Dimethylimidazolidin-2-on und 1,3-Dimethyl-3,4,5,6-tetrahydro-2(1H)-pyrimidinon.

Die Reaktion wird dabei bevorzugt in einem Temperaturbereich zwischen 0°C und dem Siedepunkt des Lösungsmittels bzw. Lösungsmittelgemisches durchgeführt.

Die Gegenwart eines Reaktionskatalysators kann von Vorteil sein. Als Katalysatoren kommen dabei starke organische und anorganische Säuren sowie Lewissäuren in Frage. Beispiele hierfür sind unter anderem Schwefelsäure, Salzsäure, Trifluoressigsäure, p-Toluolsulfonsäure, Bortrifluorid-Etherat und Titan(IV)-Alkoholate.

Verbindungen der Formel VI können in enantiomerenreiner Form erhalten werden, indem man von enantiomerenreiner Verbindungen der Formel IV ausgeht und sie in beschriebener Weise mit Verbindungen der Formel V umsetzt.

Weiterhin kann man enantiomerenreine Verbindungen der Formel VI erhalten, indem man mit racemischen bzw. diastereomeren Verbindungen der Formel VI eine klassische Racematspaltung mit geeigneten enantiomerenreinen Basen wie z.B. Brucin, Strychnin, Quinin, Quinidin, Chinchonidin, Chinchonin, Yohimbin, Morphin, Dehydroabietylamin, Ephedrin (-), (+), Deoxyephedrin (+), (-), threo-2-Amino-1-(p-nitrophenyl)-1,3-propanediol (+), (-), threo-2-(N,N-Dimethylamino)-1-(p-nitrophenyl)-1,3-propanediol (+), (-) threo-2-Amino-1-phenyl-1,3-propanediol (+), (-), α-Methylbenzylamin (+), (-), α-(1-Naphthyl)ethylamin (+), (-), α-(2-Naphthyl)ethylamin (+), (-), Aminomethylpinon, N,N-Dimethyl-1-phenylethylamin, N-Methyl-1-phenylethylamin, 4-Nitrophenylethylamin, Pseudoephedrin, Norephedrin, Norpseudoephedrin, Aminosäurederivate, Peptidderivate durchführt.

Die erfindungsgemäßen Verbindungen der allgemeinen Formel I können beispielsweise derart hergestellt werden, daß man die Carbonsäurederivate der allgemeinen Formel VI, in denen die Substituenten die angegebene Bedeutung haben, mit Verbindungen der allgemeinen Formel VII, 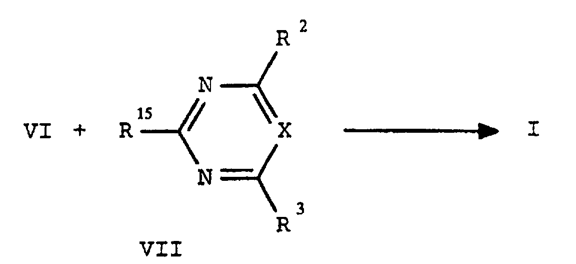 in der R¹⁵ Halogen oder R¹⁶-SO₂- bedeutet, wobei R¹⁶ C₁-C₄-Alkyl, C₁-C₄-Halogenalkyl oder Phenyl sein kann, zur Reaktion bringt. Die Reaktion findet bevorzugt in einem der oben genannten inerten Verdünnungsmittel unter Zusatz einer geeigneten Base, d.h. einer Base, die eine Deprotonierung des Zwischenproduktes VI bewirkt, in einem Temperaturbereich von Raumtemperatur bis zum Siedepunkt des Lösungsmittels statt.

Verbindungen der Formel VII sind bekannt, teilweise käuflich oder können nach allgemein bekannter Weise hergestellt werden.

Als Base kann ein Alkali- oder Erdalkalimetallhydrid wie Natriumhydrid, Kaliumhydrid oder Calciumhydrid, ein Carbonat wie Alkalimetallcarbonat, z.B. Natrium- oder Kaliumcarbonat, ein Alkali- oder Erdalkalimetallhydroxid wie Natrium- oder Kaliumhydroxid, eine metallorganische Verbindung wie Butyllithium oder ein Alkaliamid wie Lithiumdiisopropylamid oder Lithiumamid dienen.

Verbindungen der Formel I können auch dadurch hergestellt werden, daß man von den entsprechenden Carbonsäuren, d. h. Verbindungen der Formel I, in denen R¹ Hydroxyl bedeutet, ausgeht und diese zunächst auf übliche Weise in eine aktivierte Form wie ein Halogenid, ein Anhydrid oder Imidazolid überführt und dieses dann mit einer entsprechenden Hydroxylverbindung HOR¹⁰ umsetzt. Diese Umsetzung läßt sich in den üblichen Lösungsmitteln durchführen und erfordert oft die Zugabe einer Base, wobei die oben genannten in Betracht kommen. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Außerdem können Verbindungen der Formel I auch dadurch hergestellt werden, daß man von den Salzen der entsprechenden Carbonsäuren ausgeht, d. h. von Verbindungen der Formel I, in denen R für eine Gruppe COR¹ und R¹ für OM stehen, wobei M ein Alkalimetallkation oder das Äquivalent eines Erdalkalimetallkations sein kann. Diese Salze lassen sich mit vielen Verbindungen der Formel R¹-A zur Reaktion bringen, wobei A eine übliche nucleofuge Abgangsgruppe bedeutet, beispielsweise Halogen wie Chlor, Brom, Iod oder gegebenenfalls durch Halogen, Alkyl oder Halogenalkyl substituiertes Aryl- oder Alkylsulfonyl wie z.B. Toluolsulfonyl und Methylsulfonyl oder eine andere äquivalente Abgangsgruppe. Verbindungen der Formel R¹-A mit einem reaktionsfähigen Substituenten A sind bekannt oder mit dem allgemeinen Fachwissen leicht zu erhalten. Diese Umsetzung läßt sich in den üblichen Lösungsmitteln durchführen und wird vorteilhaft unter Zugabe einer Base, wobei die oben genannten in Betracht kommen, vorgenommen.

Der Rest R in Formel I ist breit variabel. Beispielsweise steht R für eine Gruppe 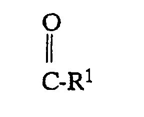 in der R¹ die folgende Bedeutung hat:
a) Wasserstoff;
b) eine Succinylimidoxygruppe;
c) ein über ein Stickstoffatom verknüpfter 5-gliedriger Heteroaromat wie Pyrrolyl, Pyrazolyl, Imidazolyl und Triazolyl, welcher ein bis zwei Halogenatome, insbesondere Fluor und Chlor und/oder ein bis zwei der folgenden Reste tragen kann:
   C₁-C₄-Alkyl wie Methyl, Ethyl, 1-Propyl, 2-Propyl, 2-Methyl-2-propyl, 2-Methyl-1-propyl, 1-Butyl, 2-Butyl;
   C₁-C₄-Halogenalkyl, insbesondere C₁-C₂-Halogenalkyl wie beispielsweise Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl;
   C₁-C₄-Halogenalkoxy, insbesondere C₁-C₂-Halogenalkoxy wie Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;
   C₁-C₄-Alkoxy wie Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy;
   C₁-C₄-Alkylthio wie Methylthio, Ethylthio, Propylthio, 1-Methylethylthio, Butylthio, 1-Methylpropylthio, 2-Methylpropylthio, 1,1-Dimethylethylthio, insbesondere Methylthio und Ethylthio;
d) R¹ ferner ein Rest 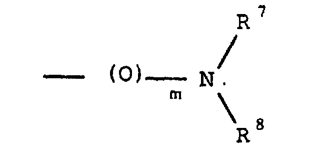 in dem m für 0 oder 1 steht und R⁷ und R⁸, die gleich oder unterschiedlich sein können, die folgende Bedeutung haben:
   Wasserstoff
   C₁-C₈-Alkyl, insbesondere C₁-C₄-Alkyl wie oben genannt;
   C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl;
   C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl
   C₃-C₈-Cycloalkyl, wie Cyclopropyl, Cyclobutyl, Cyclopentyl, Cyclohexyl und Cycloheptyl, Cyclooctyl, wobei diese Alkyl-, Cycloalkyl-, Alkenyl- und Alkinylgruppen jeweils ein bis fünf Halogenatome, insbesondere Fluor oder Chlor und/oder ein bis zwei der folgenden Gruppen tragen können:
   C₁-C₄-Alkyl, C₁-C₄-Alkoxy, C₁-C₄-Alkylthio, C₁-C₄-Halogenalkoxy wie vorstehend genannt, C₃-C₆-Alkenyloxy, C₃-C₆-Alkenylthio, C₃-C₆-Alkinyloxy, C₃-C₆-Alkinylthio, wobei die in diesen Resten vorliegenden Alkenyl- und Alkinylbestandteile vorzugsweise den oben genannten Bedeutungen entsprechen;
   C₁-C₄-Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl;
   C₁-C₄-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl;
   C₃-C₆-Alkenylcarbonyl, C₃-C₆-Alkinylcarbonyl, C₃-C₆-Alkenyloxycarbonyl und C₃-C₆-Alkinyloxycarbonyl, wobei die Alkenyl- bzw. Alkinylreste vorzugsweise, wie voranstehend im einzelnen aufgeführt, definiert sind;
   Phenyl, gegebenenfalls ein- oder mehrfach, z.B. einbis dreifach substituiert durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio wie beispielsweise 2-Fluorphenyl, 3-Chlorphenyl, 4-Bromphenyl, 2-Methylphenyl, 3-Nitrophenyl, 4-Cyanophenyl, 2-Trifluormethylphenyl, 3-Methoxyphenyl, 4-Trifluorethoxyphenyl, 2-Methylthiophenyl, 2,4-Dichlorphenyl, 2-Methoxy-3-methylphenyl, 2,4-Dimethoxyphenyl, 2-Nitro-5-cyanophenyl, 2,6-Difluorphenyl;
   Di-C₁-C₄-Alkylamino wie insbesondere Dimethylamino, Dipropylamino, N-Propyl-N-methylamino, N-Propyl-N-ethylamino, Diisopropylamino, N-Isopropyl-N-methylamino, N-Isopropyl-N-ethylamino, N-Isopropyl-N-propylamino;
   R⁷ und R⁸ ferner Phenyl, das durch einen oder mehrere, z.B. ein bis drei der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio, wie insbesondere oben genannt;
   oder R⁷ und R⁸ bilden gemeinsam eine zu einem Ring geschlossene, optionell substituierte, z.B. durch C₁-C₄-Alkyl substituierte C₄-C₇-Alkylenkette, die ein Heteroatom, ausgewählt aus der Gruppe Sauerstoff, Schwefel oder Stickstoff, enthalten kann wie -(CH₂)₄-, -(CH₂)₅-, -(CH₂)₆-, -(CH₂)₇-, -(CH₂)₂-O-(CH₂)₂-, -CH₂-S-(CH₂)₃-, -(CH₂)₂-O-(CH₂)₃-, -NH-(CH₂)₃-, -CH₂-NH-(CH₂)₂-, -CH₂-CH=CH-CH₂-, -CH=CH-(CH₂)₃-;
e) R¹ ferner eine Gruppe 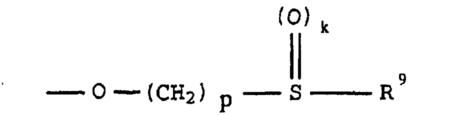 in der k die Werte 0, 1 und 2, p die Werte 1, 2, 3 und 4 annehmen und R⁹ für
   C₁-C₄-Alkyl, C₁-C₄-Halogenalkyl, C₃-C₆-Alkenyl, C₃-C₆-Alkinyl oder gegebenenfalls substituiertes Phenyl steht, wie insbesondere oben genannt.
f) R¹ ferner ein Rest OR¹⁰, worin R¹⁰ bedeutet:
   Wasserstoff, das Kation eines Alkalimetalls wie Lithium, Natrium, Kalium oder das Kation eines Erdalkalimetalls wie Calcium, Magnesium und Barium oder ein umweltverträgliches organisches Ammoniumion wie tertiäres C₁-C₄-Alkylammonium oder das Ammoniumion;
   C₃-C₈-Cycloalkyl wie vorstehend genannt, welches ein bis drei C₁-C₄-Alkylgruppen tragen kann;
   C₁-C₈-Alkyl wie insbesonder Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, welches ein bis fünf Halogenatome, insbesondere Fluor und Chlor und/oder einen der folgenden Reste tragen kann:
   C₁-C₄-Alkoxy, C₁-C₄-Alkylthio, Cyano, C₁-C₄-Alkylcarbonyl, C₃-C₈-Cycloakyl, C₁-C₄-Alkoxycarbonyl, Phenyl, Phenoxy oder Phenylcarbonyl, wobei die aromatischen Reste ihrerseits jeweils ein bis fünf Halogenatome und/oder ein bis drei der folgenden Reste tragen können: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
   eine C₁-C₈-Alkylgruppe wie vorstehend genannt, welch ein bis fünf Halogenatome, insbesonder Fluor und/oder Chlor tragen kann und einen der folgenden Reste trägt: ein 5-gliedriger Heteroaromat, enthaltend ein bis drei Stickstoffatome, oder ein 5-gliedriger Heteroaromat enthaltend ein Stickstoffatom und ein Sauerstoff- oder Schwefelatom, welcher ein bis vier Halogenatome und/oder ein bis zwei der folgenden Reste tragen kann:
   Nitro, Cyano, C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3-Isopropylisoxazol-5-yl, 3-Methylisoxazol-5-yl, Oxazol-2-yl, Thiazol-2-yl, Imidazol-2-yl, 3-Ethylisoxazol-5-yl, 3-Phenylisoxazol-5-yl, 3-tert.-Butylisoxazol-5-yl;
   eine C₂-C₆-Alkylgrupe, welche in der 2-Position einen der folgenden Reste trägt: C₁-C₄-Alkoxyimino, C₃-C₆-Alkinyloxyimino, C₃-C₆-Halogenalkenyloxyimino oder Benzyloxyimino;
   eine C₃-C₆-Alkenyl- oder eine C₃-C₆-Alkinylgruppe, wobei diese Gruppen ihrerseits ein bis fünf Halogenatome tragen können;
   R¹⁰ ferner ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
   ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-1-pyrazolyl, 3-Phenyl-1-pyrazolyl, 4-Phenyl-1-pyrazolyl, 4-Chlor-1-pyrazol 4-Brom-1-pyrazolyl 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3,4-Dichlorimidazol-1-yl;
   R¹⁰ ferner ein Gruppe 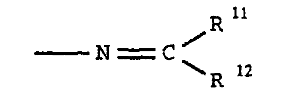 worin R¹¹ und R¹², die gleich oder verschieden sein können, bedeuten:
   C₁-C₈-Alkyl, C₃-C₆-Alkenyl, C₃-C₆-Alkinyl, C₃-C₈-Cycloalkyl, wobei diese Reste einen C₁-C₄-Alkoxy, C₁-C₄-Alkylthio und/oder einen gegebenenfalls substituierten Phenylrest, wie insbesondere vorstehend genannt, tragen können;
   Phenyl, das durch einen oder mehrere, z.B. einen bis drei der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio, wobei diese Reste insbesondere den oben genannten entsprechen;
   oder R¹¹ und R¹² bilden gemeinsam eine C₃-C₁₂-Alkylenkette, welche ein bis drei C₁-C₄-Alkylgruppen tragen und ein Heteroatom aus der Gruppe Sauerstoff, Schwefel und Stickstoff enthalten kann, wie insbesondere bei R⁷ und R⁸ genannt.
g) R¹ ferner ein Rest 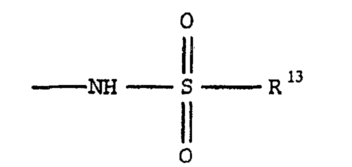 worin R¹³ bedeutet:
   C₁-C₄-Alkyl, C₃-C₆-Alkenyl, C₃-C₆-Alkinyl, C₃-C₈-Cycloalkyl wie insbesondere vorstehend genannt, wobei diese Reste einen C₁-C₄-Alkoxy-, C₁-C₄-Alkylthio- und/oder einen Phenylrest wie oben genannt tragen können;
   Phenyl, gegebenenfalls substituiert, insbesondere wie vorstehend genannt.
h) R¹ ein Rest 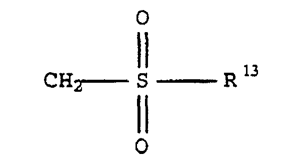 worin R¹³ die oben genannte Bedeutung hat.
   - R: kann weiterhin sein:
   Tetrazol oder Nitril.

Im Hinblick auf die biologische Wirkung sind Carbonsäurederivate der allgemeinen Formel I - sowohl als reine Enantiomere bzw. reine Diastereomere oder als deren Mischung - bevorzugt, in denen die Substituenten folgende Bedeutung haben:
- R²: die bei R¹ im einzelnen genannten C₁-C₄-Alkyl-, C₁-C₄-Halogenalkyl-, C₁-C₄-Alkoxy-, C₁-C₄-Halogenalkoxy-, C₁-C₄-Alkylthiogruppen und Halogenatome, insbesondere Chlor, Methyl, Methoxy, Ethoxy, Difluormethoxy, Trifluormethoxy;
- X: Stickstoff oder CR¹⁴, worin
- R¹⁴: Wasserstoff oder Alkyl bedeutet oder CR¹⁴ zusammen mit CR³ einen 4- bis 5-gliedrigen Alkylen- oder Alkenylenring bildet, in der jeweils eine Methylengruppe durch Sauerstoff oder Schwefel ersetzt sein kann wie -CH₂-CH₂-O-, -CH=CH-O-, -CH₂-CH₂-CH₂-O-, -CH=CH-CH₂O-, insbesondere Wasserstoff, -CH₂-CH₂-O-, -CH(CH₃)-CH(CH₃)-O-, -C(CH₃)=C(CH₃)-O-, -CH=C(CH₃)-O- oder -C(CH₃)=C(CH₃)-S;
- R³: die bei R¹ genannten C₁-C₄-Alkyl-, C₁-C₄-Halogenalkyl-, C₁-C₄-Alkoxy-, C₁-C₄-Halogenalkoxy-, C₁-C₄-Alkylthiogruppen und Halogenatome, insbesondere Chlor, Methyl, Methoxy, Ethoxy, Difluormethoxy, Trifluormethoxy oder mit R¹⁴ wie oben genannt zu einem 5- oder 6-gliedrigen Ring verknüpft ist;
- R⁴ und R⁵: Phenyl oder Naphthyl, die durch einen oder mehrere, z.B. einen bis drei der folgenden Reste substituiert sein können: Halogen, Nitro, Cyano, Hydroxy, Mercapto, Amino, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino, Di-C₁-C₄-alkylamino, C₁-C₄-Alkylcarbonyl, C₁-C₄-Alkoxycarbonyl;
Phenyl oder Naphthyl, die orthoständig über eine direkte Bindung, eine Methylen-, Ethylen- oder Ethenylengruppe, ein Sauerstoff- oder Schwefelatom oder eine SO₂-, NH- oder N-Alkyl-Gruppe miteinander verbunden sind oder C₃-C₇-Cycloalkyl;
- R⁶: C₁-C₄-Alkyl, C₃-C₅-Alkenyl, wobei die Reste jeweils ein- oder zweifach substituiert sind durch Hydroxy, Marcapto, Carboxy Cyano, oder C₁-C₄-Alkoxy;
- Z: Schwefel oder Sauerstoff,
mit der Maßgabe, dass Carbonsäurederivate der Formel I ausgeschlossen sind, wenn der Rest R COOH, der Rest R² OMe, die Reste R⁴ und R⁵ Phenyl, und die Reste Y und Z O sind, und
(1) gleichzeitig der Rest R⁶ CH₂-CH₂-SO₂-CH(CH₃)₂, der Rest R³ OMe und der Rest X CH sind, oder
(2) gleichzeitig der Rest R⁶ CH₂-CH₂-SO₂-CH(CH₃)₂, der Rest R³ NH-OCH₃ und der Rest X CH sind, oder
(3) gleichzeitig der Rest R⁶ OH-CH₂-CH₂, der Rest R³ OMe und der Rest X CH sind, oder
(4) gleichzeitig der Rest R⁶ HOOC-(CH₂)₂-, der Rest R³ OMe und der Rest X CH sind, oder
(5) gleichzeitig der Rest R⁶ OH-CH₂-CH₂ und die Reste R³ und X zusammen einen Rest CH₂-CH₂-CH₂-C bilden sind.

Besonders bevorzugt sind Verbindungen der Formel I - sowohl als reine Enantiomere bzw. reine Diastereomere oder als deren Mischung - in denen die Substituenten folgende Bedeutung haben:
- R²: C₁-C₄-Alkyl, C₁-C₄-Alkoxy
- X: Stickstoff oder CR¹⁴, worin
- R¹⁴: Wasserstoff oder Alkyl bedeutet oder CR¹⁴ zusammen mit CR³ einen 4- bis 5'-gliedrigen Alkylen- oder Alkenylenring bildet wie z.B. -CH₂-CH₂-CH₂-, -CH=CH-CH₂-, in der jeweils eine Methylengruppe durch Sauerstoff oder Schwefel ersetzt sein kann wie -CH₂-CH₂-O-, -CH=CH-O-, -CH₂-CH₂-CH₂-O-, -CH=CH-CH₂O-, insbesondere Wasserstoff,
-CH₂-CH₂-O-, -CH(CH₃)-CH(CH₃)-O-, -C(CH₃)=C(CH₃)-O-, -CH=C(CH₃)-O- oder -C(CH₃)=C(CH₃)-S;
- R³: die bei R¹ genannten C₁-C₄-Alkyl-, C₁-C₄-Alkoxy, C₁-C₄-Alkylthiogruppen oder mit R¹⁴ wie oben genannt zu einem 5- oder 6-gliedrigen Ring verknüpft ist;
- R⁴ und R⁵: Phenyl (gleich oder verschieden), die durch einen oder mehrere, z.B. einen bis drei der folgenden Reste substituiert sein können: Halogen, Nitro, Hydroxy, C₁-C₄-Alkyl, C₁-C₄-Alkoxy, C₁-C₄-Alkylthio oder
- R⁴ und R⁵: sind Phenylgruppen, die orthoständig über eine direkte Bindung, eine Methylen-, Ethylen- oder Ethenylengruppe, ein Sauerstoff- oder Schwefelatom oder eine SO₂-, NH- oder N-Alkyl-Gruppe miteinander verbunden sind; oder
- R⁴ und R⁵: sind C₃-C₇-Cycloalkyl;
- R⁶: C₁-C₃-Alkyl, C₃-C₄-Alkenyl, wobei die Reste jeweils ein- oder zweifach substituiert sind durch Hydroxy oder einfach substituiert sind durch Carboxy;
- Z: Schwefel oder Sauerstoff, unter der gleichen Maßgabe.

Die Verbindungen der vorliegenden Erfindung bieten ein neues therapeutisches Potential für die Behandlung von Hypertonie, pulmonalem Hochdruck, Myokardinfarkt, Angina Pectoris, akutem Nierenversagen, Niereninsuffizienz, zerebralen Vasospasmen, zerebraler Ischämie, Subarachnoidalblutungen, Migräne, Asthma, Atherosklerose, endotoxischem Schock, Endotoxin-induziertem Organversagen, intravaskulärer Koagulation, Restenose nach Angioplastie, benigne Prostata-Hyperplasie, ischämisches und durch Intoxikation verursachtes Nierenversagen bzw. Hypertonie und Krebserkrankungen, insbesondere Prostata- und Hautkrebs.

Ein weiterer Gegenstand der Erfindung ist die Kombination von Verbindungen der Formel I mit Hemmstoffen des Renin-Angiotensin-Systems (RAS). RAS-Hemmstoffe sind beispielsweise aus EP 634 175 bekannt.

Die erfindungsgemäßen Kombinationen eignen sich zur Behandlung von solchen Krankheiten, für die auch die Verbindungen der Formel I allein Wirksamkeit zeigen, insbesondere zur Behandlung von Hypertomie und chronischer Herzinsuffizienz.

Die gute Wirkung der Verbindungen läßt sich in folgenden Versuchen zeigen:

### Rezeptorbindungsstudien

Für Bindungsstudien wurden klonierte humane ET_{A}-Rezeptorexprimierende CHO-Zellen und Meerschweinchen-Kleinhirnmembranen mit > 60 % ET_{B}- im Vergleich zu ET_{A}-Rezeptoren eingesetzt.

### Membranpräparation

Die ET_{A}-Rezeptor-exprimierenden CHO-Zellen wurden in F₁₂-Medium mit 10 % fötalem Kälberserum, 1 % Glutamin, 100 E/ml Penicillin und 0,2 % Streptomycin (Gibco BRL, Gaithersburg, MD, USA) vermehrt. Nach 48 h wurden die Zellen mit PBS gewaschen und mit 0,05 % trypsinhaltiger PBS 5 min inkubiert. Danach wurde mit F₁₂-Medium neutralisiert und die Zellen durch Zentrifugation bei 300 x g gesammelt. Zur Lyse der Zellen wurde kurz das Pellet mit Lysispuffer (5 mM Tris-HCl, pH 7,4 mit 10 % Glycerin) gewaschen und danach in einer Konzentration von 10⁷-Zellen/ml Lysispuffer 30 min bei 4°C inkubiert. Die Membranen wurden bei 20.000 x g 10 min zentrifugiert und das Pellet in flüssigem Stickstoff gelagert.

Meerschweinchenkleinhirne wurden im Potter-Elvejhem-Homogenisator homogenisiert und durch differentielle Zentrifugation 10 min bei 1.000 x g und wiederholte Zentrifugation des Überstandes 10 min bei 20.000 x g gewonnen.

### Bindungstests

Für den ET_{A}- und ET_{B}-Rezeptorbindungstest wurden die Membranen in Inkubationspuffer (50 mM Tris-HCl, pH 7,4 mit 5 mM MnCl₂, 40 µg/ml Bacitracin und 0,2 % BSA) in einer Konzentration von 50 µg Protein pro Testansatz suspendiert und bei 25°C mit 25 pM [125J]-ET₁ (ET_{A}-Rezeptortest) oder 25 pM [125J]-RZ₃ (ET_{B}-Rezeptortest) in Anwesenheit und Abwesenheit von Testsubstanz inkubiert. Die unspezifische Bindung wurde mit 10⁻⁷ M ET₁ bestimmt. Nach 30 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 mit 0,2 % BSA gewaschen.

Die auf den Filtern gesammelte Radioaktivität wurde mit einem Packard 2200 CA Flüssigkeitszintillationszähler quantifiziert.

Funktionelles in vitro-Testsystem für die Suche nach Endothelinrezeptor (Subtyp A)-Antagonisten

Dieses Testsystem ist ein funktioneller, auf Zellen basierender Test für Endothelinrezeptoren. Bestimmte Zellen zeigen, wenn sie mit Endothelin 1 (ET1) stimuliert werden, einen Anstieg der intrazellulären Calciumkonzentration. Dieser Anstieg kann in intakten Zellen, die mit Calcium-sensitiven Farbstoffen beladen wurden, gemessen werden.

Aus Ratten isolierte 1-Fibroblasten, bei denen ein endogener Endothelinrezeptor vom A-Subtyp nachgewiesen wurde, wurden mit dem Fluoreszenzfarbstoff Fura 2-an wie folgt beladen: Nach Trypsinierung wurden die Zellen in Puffer A (120 mM NaCl, 5 mM KC1, 1,5 mM MgCl₂, 1 mM CaCl₂, 25 mM HEPES, 10 mM Glucose, pH 7,4) bis zu einer Dichte von 2 x 10⁶/ml resuspendiert und in 30 min bei 37°C im Dunkeln mit Fura 2-am (2 µM), Pluronics F-127 (0,04 %) und DMSO (0,2 %) inkubiert. Danach wurden die Zellen zweimal mit Puffer A gewaschen und zu 2 x 10⁶/ml resuspendiert.

Das Fluoreszenzsignal von 2 x 10⁵ Zellen pro ml bei Ex/Em 380/510 wurde bei 30°C kontinuierlich registriert. Zu den Zellen wurden die Testsubstanzen und nach einer Inkubationszeit von 3 min ET1 wurde die maximale Änderung der Fluoreszenz bestimmt. Die Antwort der Zellen auf ET1 ohne vorherige Zugabe einer Testsubstanz diente als Kontrolle und wurde gleich 100 % gesetzt.

### Testung der ET-Antagonisten in vivo

Männliche 250 - 300 g schwere SD-Ratten wurden mit Amobarbital narkotisiert, künstlich beatmet, vagotomisiert und despinalisiert. Die Arteria carotis und Vena jugularis wurden kathetisiert.

In Kontrolltieren führt die intravenöse Gabe von 1 µg/kg ET1 zu einem deutlichen Blutdruckanstieg, der über einen längeren Zeitraum anhält.

Den Testtieren wurde 5 min vor der ET1 Gabe die Testverbindungen i.v. injiziert (1 ml/kg). Zur Bestimmung der ET-antagonistischen Eigenschaften wurde der Blutdruckanstieg in den Testtieren mit dem in den Kontrolltieren verglichen.

### Endothelin-1 induzierter "sudden death" an Mäusen

Das Testprinzip besteht in der Hemmung des durch Endothelin verursachten plötzlichen Herztodes der Maus, der wahrscheinlich durch Verengung der Herzkranzgefäße bedingt ist, durch Vorbehandlung mit Endothelin-Rezeptorantagonisten. Nach intravenöser Injektion von 10 nmol/kg Endothelin im Volumen von 5 ml/kg Körpergewicht kommt es innerhalb weniger Minuten zum Tod der Tiere.

Die letale Endothelin-1 Dosis wird jeweils an einem kleinen Tierkollektiv überprüft. Wird die Prüfsubstanz intravenös appliziert, erfolgt meist 5 min danach die im Referenzkollektiv letale Endothelin-1 Injektion. Bei anderen Applikationsarten verlängern sich die Vorgabezeiten, gegebenenfalls bis zu mehreren Stunden.

Die Überlebensrate wird dokumentiert und effektive Dosen, die 50 % der Tiere 24 h oder länger gegen den Endothelin-Herztod schützen (ED 50) werden ermittelt.

### Funktioneller Gefäßtest für Endothelin-Rezeptorantagonisten

An Aortensegmenten des Kaninchens wird nach einer Vorspannung von 2 g und einer Relaxationszeit von 1 h in Krebs-Henseleitlösung bei 37°C und einem pH-Wert zwischen 7,3 und 7,4 zunächst eine K⁺-Kontraktur ausgelöst. Nach Auswaschen wird eine Endothelin-Dosiswirkungskurve bis zum Maximum erstellt.

Potentielle Endothelin-Antagonisten werden an anderen Präparaten des gleichen Gefäßes 15 min vor Beginn der Endothelin-Dosiswirkungskurve appliziert. Die Effekte des Endothelins werden in % der K⁺-Kontraktur berechnet. Bei wirksamen Endothelin-Antagonisten kommt es zur Rechtsverschiebung der Endothelin-Dosiswirkungskurve.

Die erfindungsgemäßen Verbindungen können in üblicher Weise oral oder parenteral (subkutan, intravenös, intramuskulär, intraperotoneal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis zwischen etwa 0,5 und 50 mg/kg Körpergewicht bei oraler Gabe und zwischen etwa 0,1 und 10 mg/kg Körpergewicht bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1991). Die so erhaltenen Applikationsformen enthalten den Wirkstoff normalerweise in einer Menge von 0,1 bis 90 Gew.-%.

### Synthesebeispiele

### Beispiel 1

### 3-(2-Acetoxy-ethoxy)-2-hydroxy-3,3-diphenyl-propionsäuremethylester

7,95 g (31,3 mmol) 3,3-Diphenyl-2,3-epoxypropionsäuremethylester wurden unter N₂ in 20 ml Diethylether gelöst, auf 0°C gekühlt und mit 5,87 ml (31,3 mmol) Essigsäure-2-hydroxyethylester (50 %ig) und 3 Tropfen BF₃·Et₂O versetzt. Nach Entfernen des Eisbades wurde 2 h bei RT nachgerührt.

Die Reaktionslösung wurde nacheinander mit NaCl-Lösung und NaHCO₃-Lösung gewaschen und die organische Phase über MgSO₄ getrocknet und eingeengt. Man erhielt 12,3 g eines schwach-gelben Öls, das ohne weitere Reinigung und Charakterisierung umgesetzt wurde.

### Beispiel 2

### 3-(2-Acetoxy-ethoxy)-2-(4-methoxy-6-methyl-pyrimidin-2-yloxy)-3,3-diphenylpropionsäuremethylester

4 g (11,1 mmol) 3-(2-Acetoxy-ethoxy)-2-hydroxy-3,3-diphenylpropionsäuremethylester wurden unter N₂ in 20 ml DMF gelöst, mit 770 mg (5,6 mmol) K₂CO₃ und 2,24 g (11,1 mmol) 2-Methansulfonyl-4-methoxy-6-methylpyrimidin versetzt und 2 h bei 80°C gerührt. Anschließend wurde mit 20 ml H₂O verdünnt, zweimal mit 30 ml Diethylether extrahiert, die organische Phase über MgSO₄ getrocknet, eingeengt und der Rückstand an Kieselgel mit Essigester/Cyclohexangemischen chromatographisch gereinigt. Man erhielt 4,8 g (90 %) eines farblosen Öls.
- ¹H-NMR (CDCl₃) δ:: 2,10 (s, 3H); 2,35 (s, 3H); 3,50 (s, 3H); 3,85 (s, 6H); 4,00 (m, 2H); 4,30 (m, 2H); 6,00 (s, 1H), 6,25 (s, 1H)7,20 - 7,50 (m, 10H)

### Beispiel 3

### 3-(2-Hydroxy-ethoxy)-2-(4-methoxy-6-methyl-pyrimidin-2-yloxy)-3,3-diphenylpropionsäure

4,8 g (10 mmol) 3-(2-Acetoxy-ethoxy)-2-(4-methoxy-6-methyl-pyrimidin-2-yloxy(3,3-diphenyl-propionsäuremethylester wurden in 80 ml Dioxan und 40 ml 1n KOH-Lösung gelöst und 8 h bei 90°C gerührt. Die Lösung wurde mit 50 ml H₂O verdünnt und mit Diethylether extrahiert. Die wäßrige Phase wurde mit 10 ml 1N HCl-Lösung neutralisiert, zweimal mit Diethylether extrahiert, die organische Phase getrocknet und eingeengt. Der Rückstand wurde an Kieselgel mit Cyclohexan/Essigestergemischen chromatographisch gereinigt und nach Auskristallisieren aus Diethylether/Hexan wurden 1,2 g (28 %) farblose Kristalle erhalten.
- ¹H-NMR (CDCl₃) δ:: 2,25 (s, 3H); 3,55 (m, 2H); 3,65-3,85 (m, 3H); 3,90 (s, 6H), 6,10 (s, 1H); 6,25 (s, 1H); 6,40 (breit, 1H), 7,20 - 7,60 (m, 10H)

### Beispiel 4

### 3-(2-Hydroxy-2-methoxycarbonyl-1,1-diphenyl-ethoxy)-2,2-dimethylpropionsäuremethylester

12,7 g (50 mmol) 3,3-Diphenyl-2,3-epoxypropionsäuremethylester wurden in 50 ml Diethylether gelöst, mit 6,6 g (50 mmol) 3-Hydroxy-2,2-dimethyl-propionsäuremethylester und 1 ml BF₃·Et₂O versetzt und 18 h bei Raumtemperatur gerührt. Das Lösungsmittel wurde abgedampft und der ölige Rückstand ohne weitere Reinigung und Charakterisierung umgesetzt.

### Beispiel 5

### 3-[2-Methoxycarbonyl-2-(4-methoxy-6-methyl-pyrimidin-2-yloxy)-1,1-diphenyl-ethoxy]2,2-dimethyl-propionsäuremethylester

10 g (25,9 mmol) 3-(2-Hydroxy-2-methoxycarbonyl-1,1-diphenylethoxy)-2,2-dimethyl-propionsäuremethylester wurden unter N₂ in 40 ml DMF gelöst, mit 1,78 g (13 mmol) K₂CO₃ und 5,2 g (25,9 mmol) 2-Methansulfonyl-4-methoxy-6-methylpyrimidin versetzt und 2 h bei 80°C gerührt. Anschließend wurde mit 40 ml H₂O verdünnt, zweimal mit 30 ml Diethylether extrahiert, die organische Phase über MgSO₄ getrocknet, eingeengt und der Rückstand an Kieselgel mit Essigester/Cyclohexangemischen chromatographisch gereinigt. Man erhielt nach Auskristallisieren aus Diethylether/Hexan 11,8 g (90 %) des Produkts als farblose Kristalle.
Smp: 143°C

### Beispiel 6

### 3-[2-Carboxy-2-(4-methoxy-6-methyl-pyrimidin-2-yloxy)-1,1-diphenyl-ethoxy]-2,2-dimethyl-propionsäure

10,1 g (20 mmol) 3-[2-Methoxycarbonyl-2-(4-methoxy-6-methyl-pyrimidin-2-yloxy)-1,1-diphenyl-ethoxy]-2,2-dimethyl-propionsäuremethylester wurden in 50 ml Dioxan und 50 ml 2N NaOH-Lösung gelöst und 4 h bei 80°C gerührt. Die Lösung wurde mit 300 ml H₂O verdünnt und mit 100 ml Essigester extrahiert. Die wäßrige Phase wurde mit 1N HCl neutralisiert, mit Essigester extrahiert, die organische Phase über MgSO₄ getrocknet, filtriert und eingeengt. Der ölige Rückstand wurde aus Diethylether/Hexan auskristallisiert und man erhielt 4,1 g (42 %) farblose Kristalle.
- ¹H-NMR (CDCl₃) δ:: 1,10 (s, 3H); 1,20 (s, 3H); 2,50 (s, 3H); 3,65 (d, 1H); 3,80 (s, 3H); 3,90 (d, 1H); 5,95 (s, 1H); 6,25 (s, 1H); 7,20 - 7,50 (m, 10H)

Analog lassen sich die in Tabelle 1 aufgeführten Verbindungen herstellen.

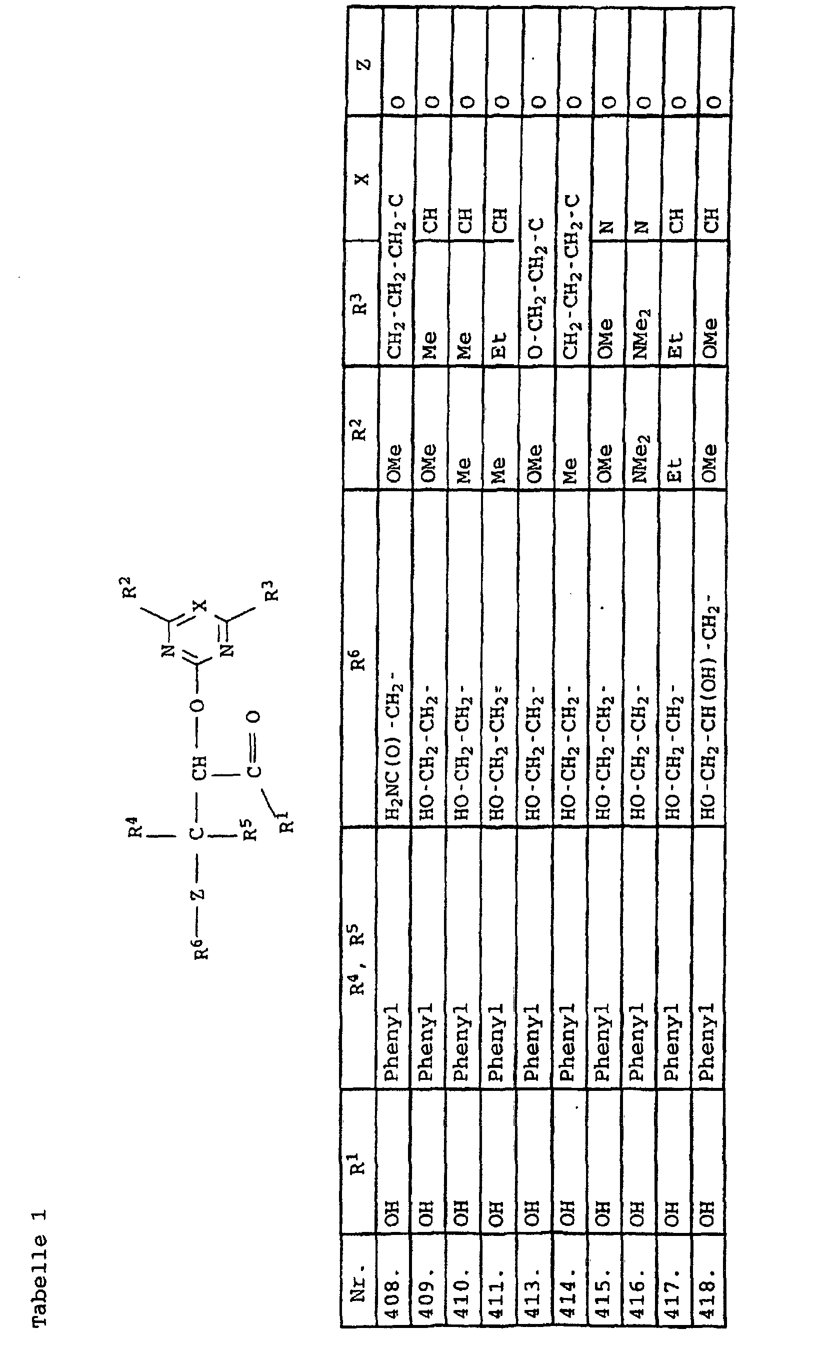

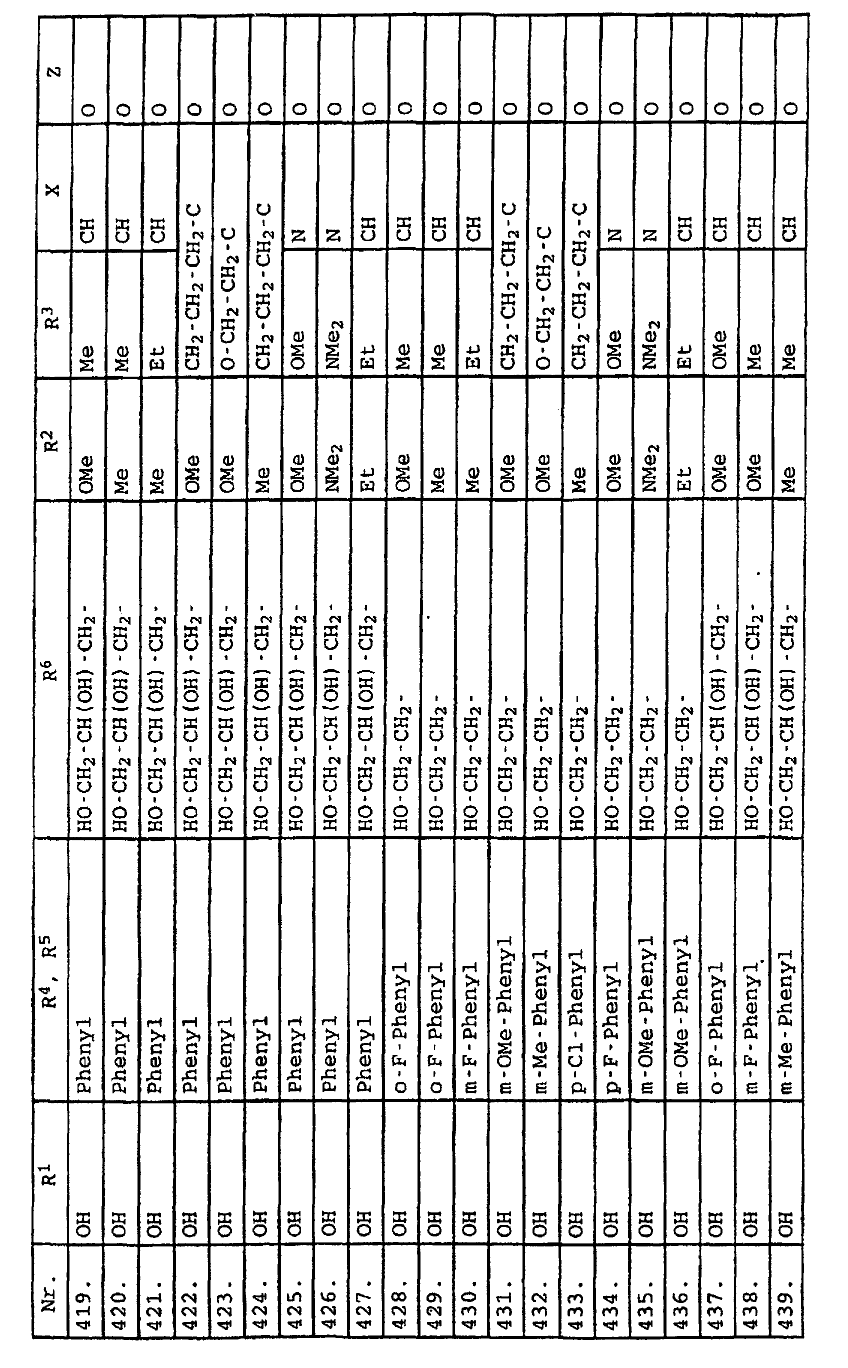

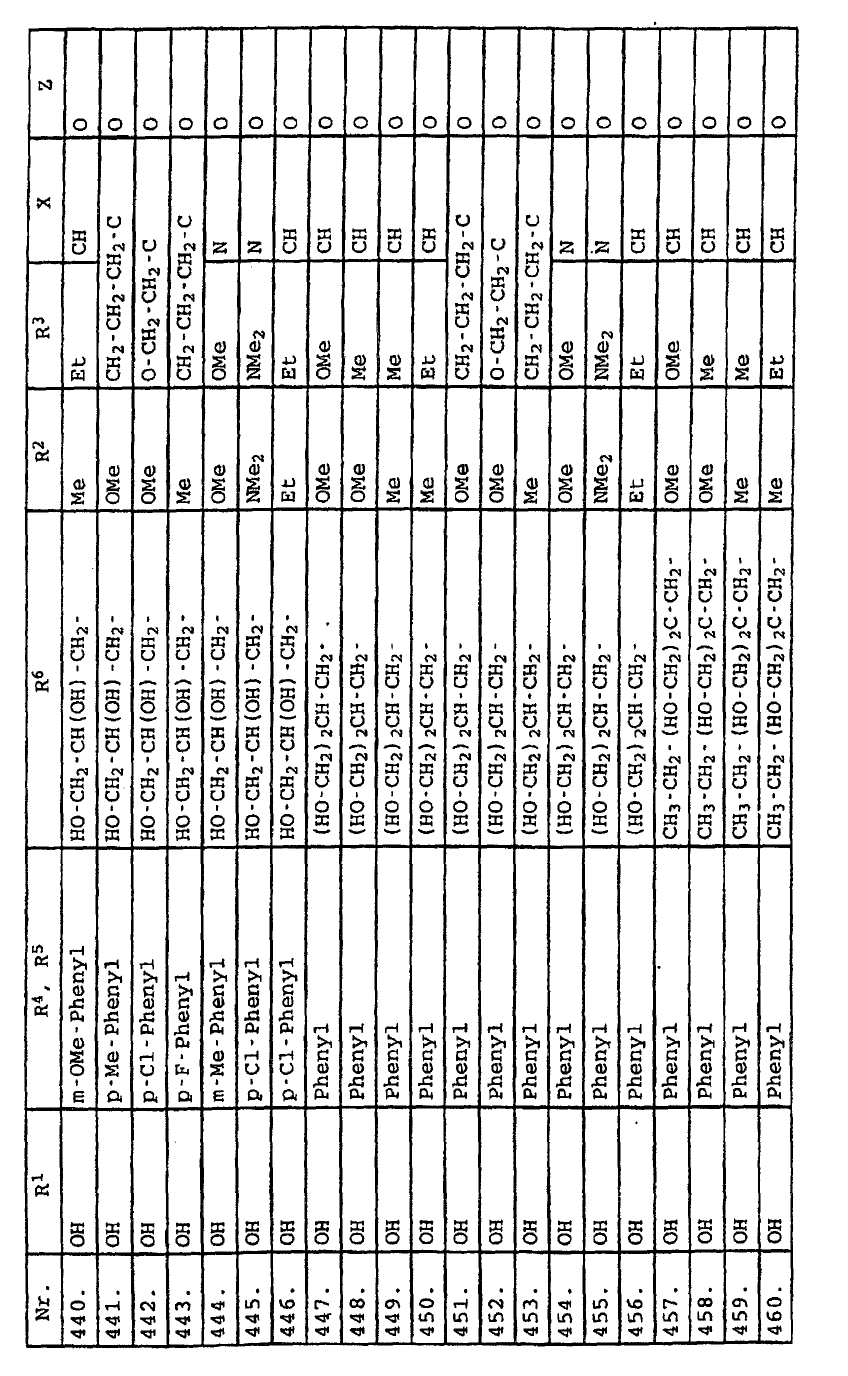

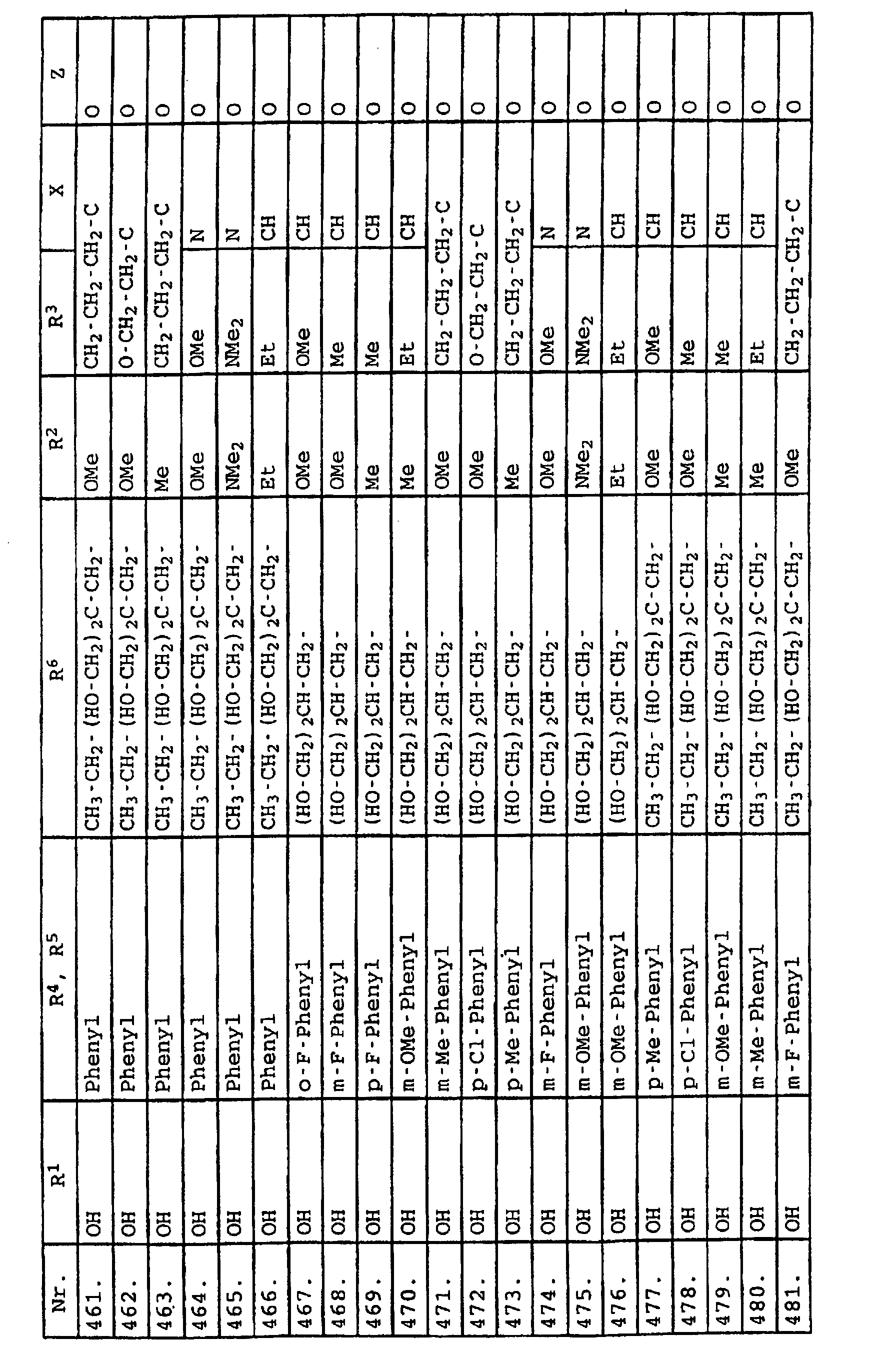

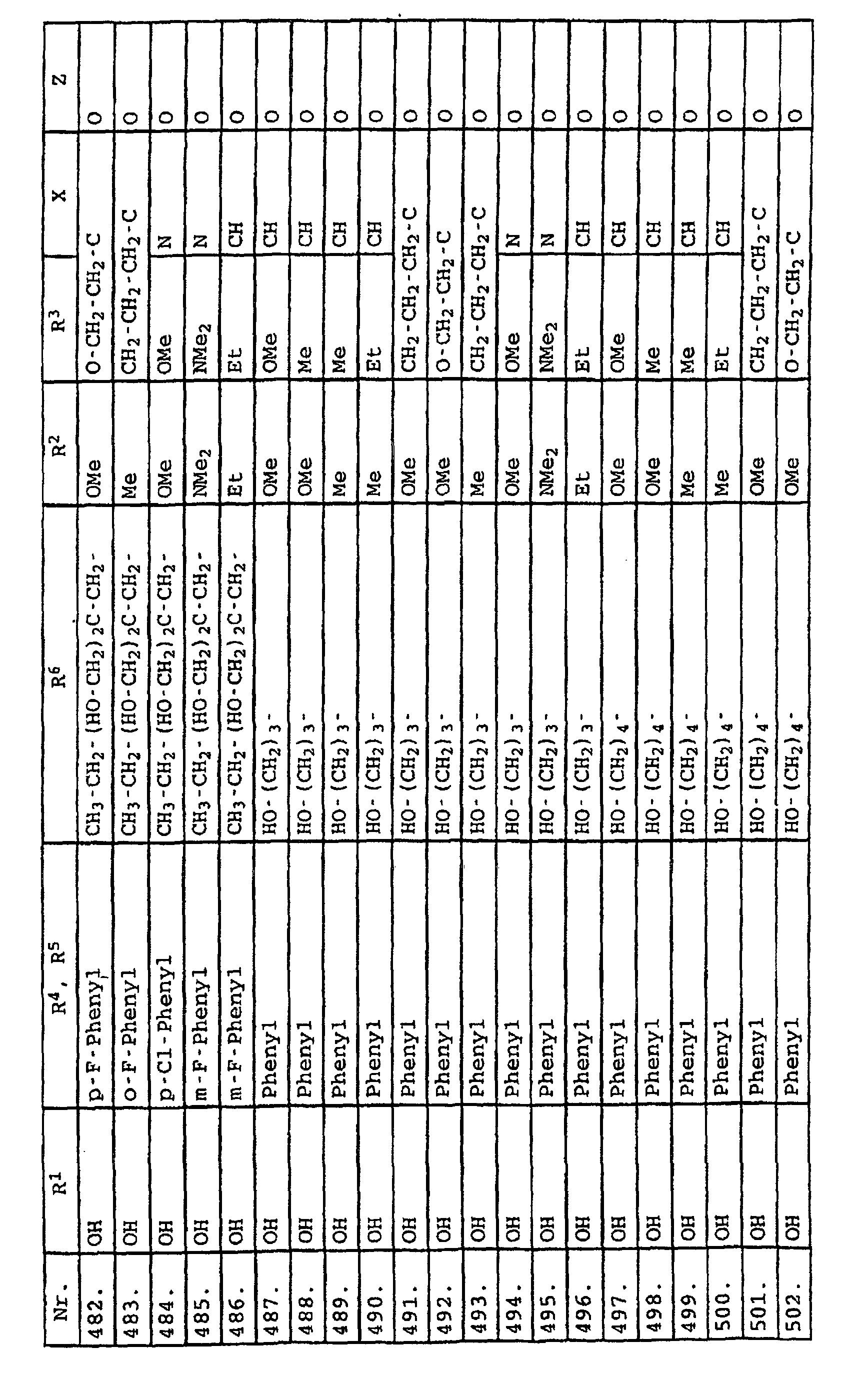

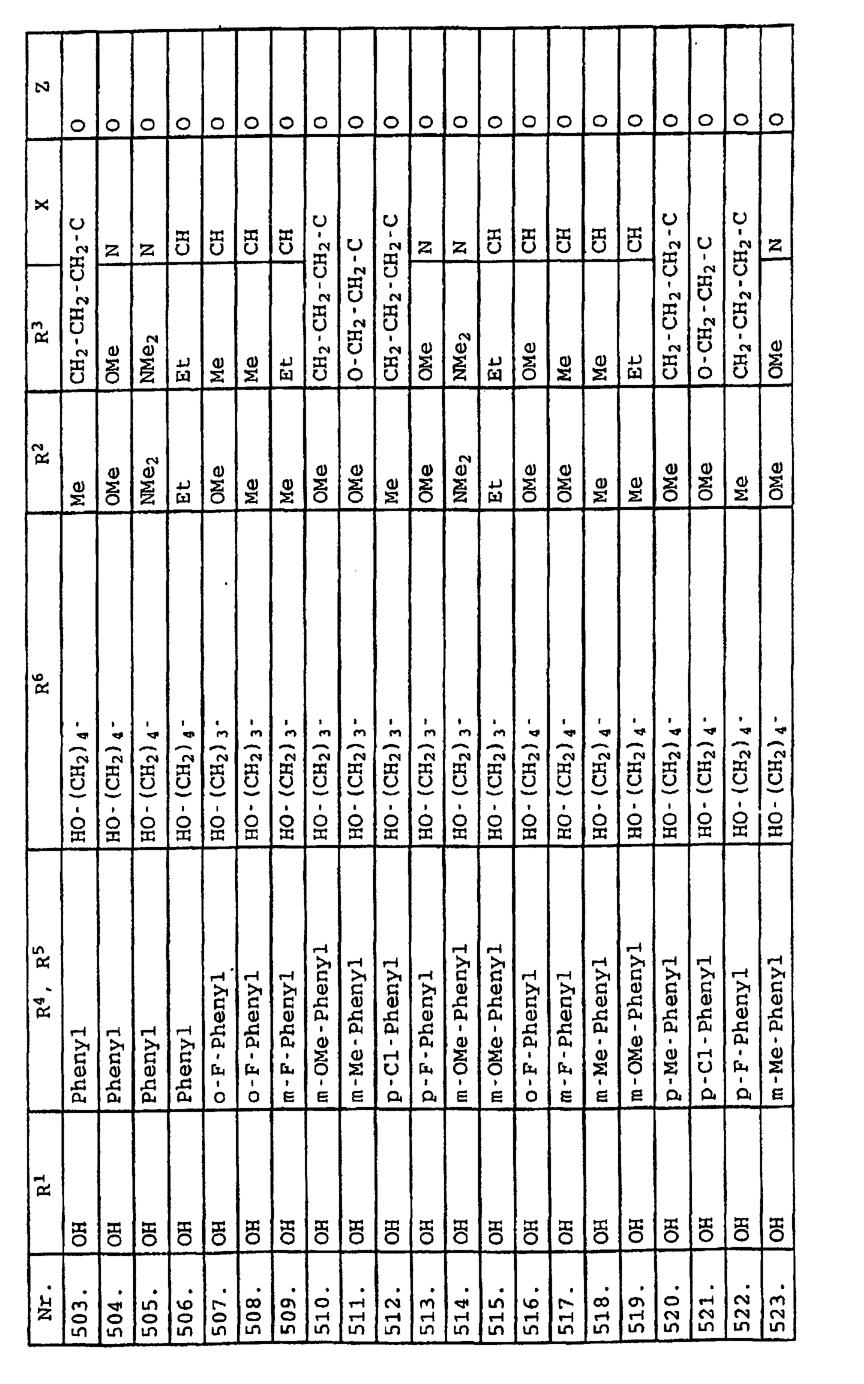

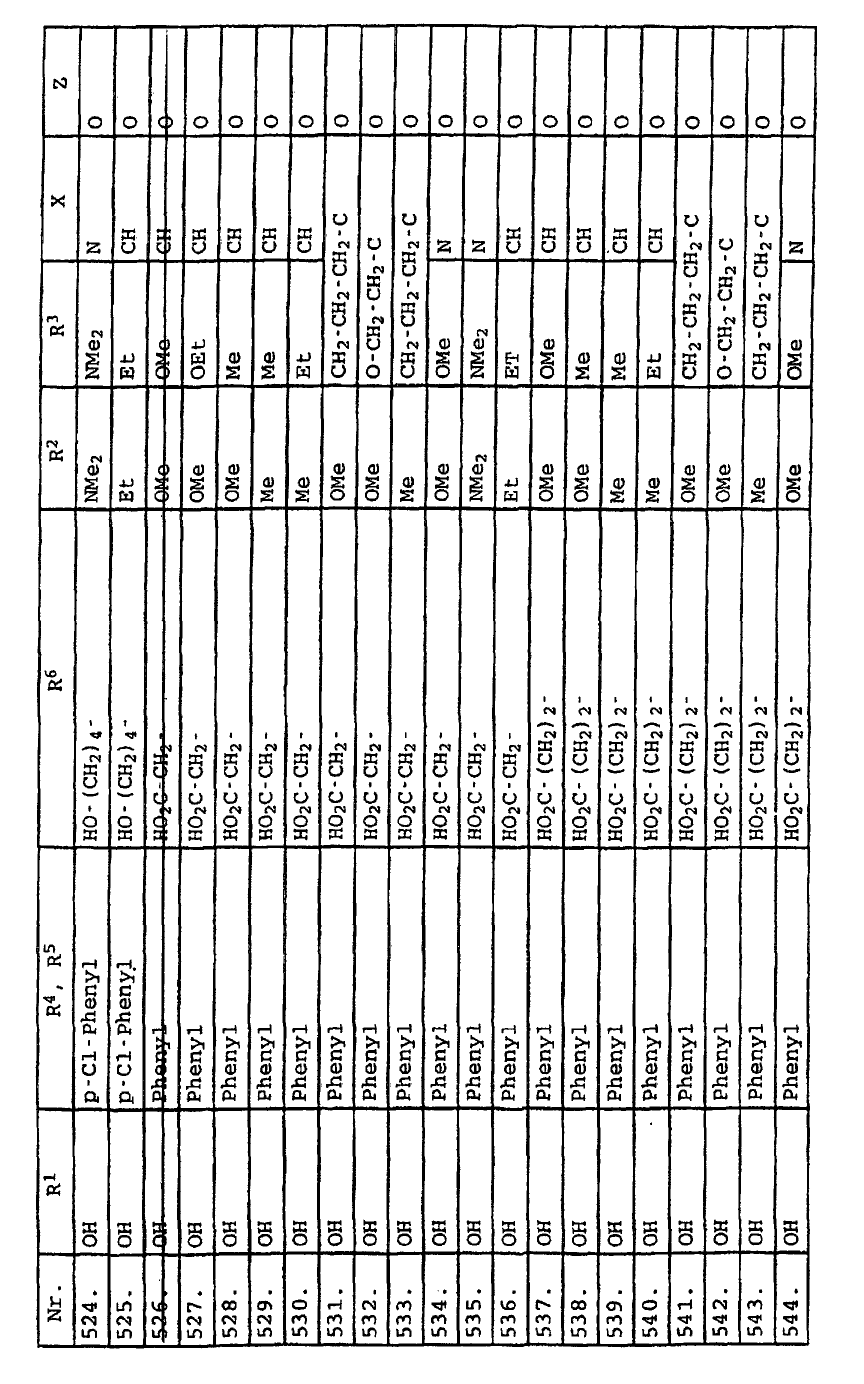

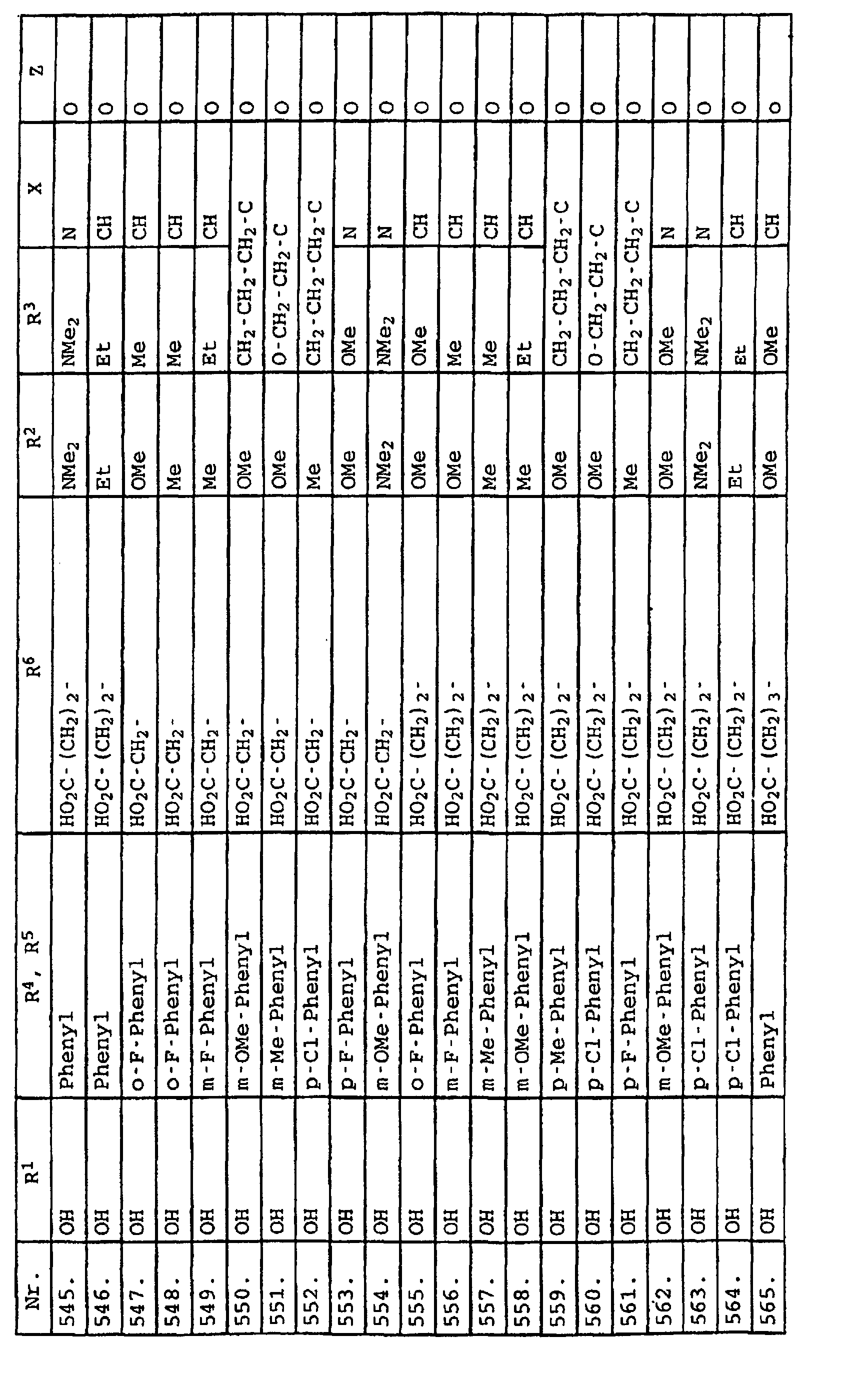

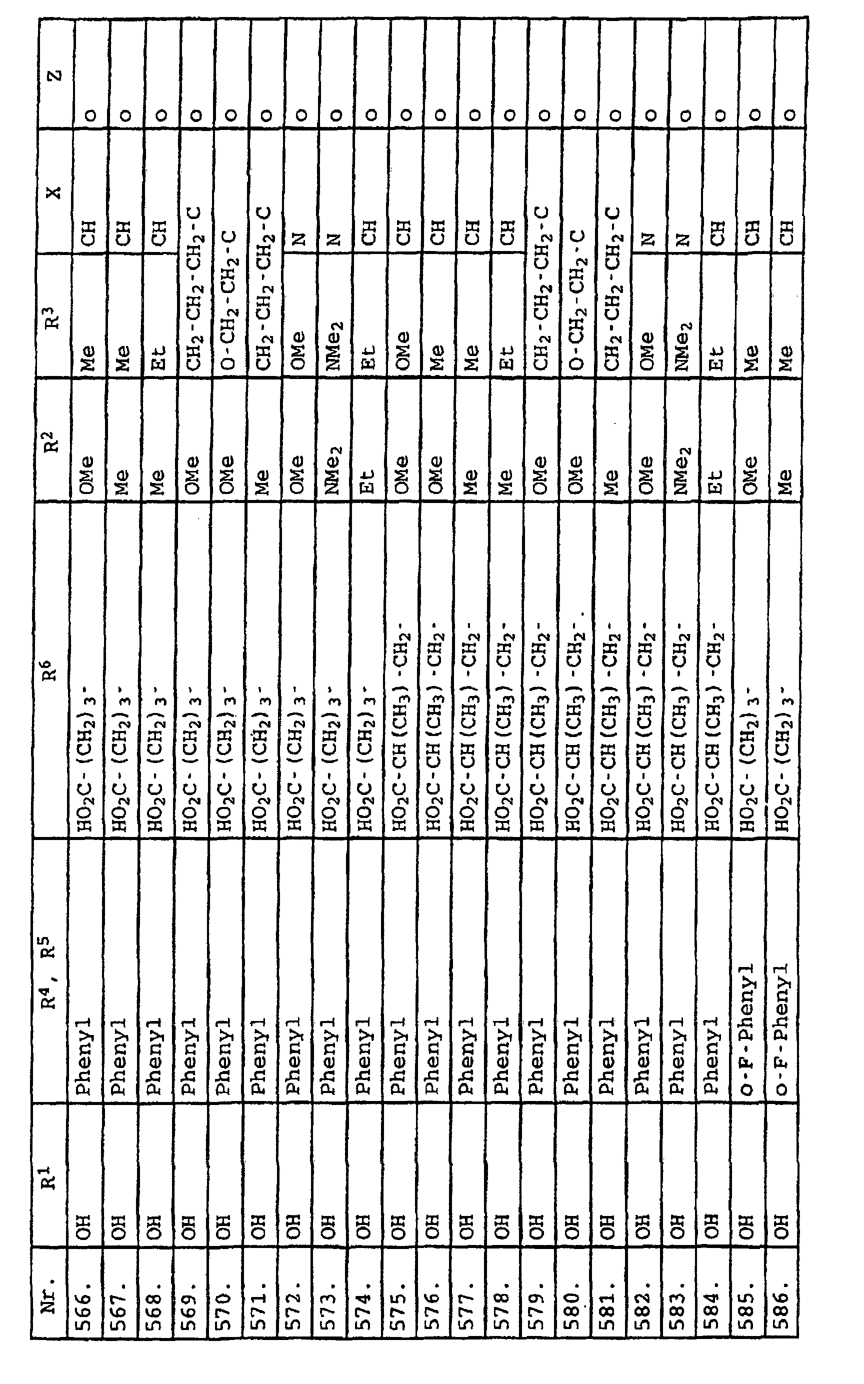

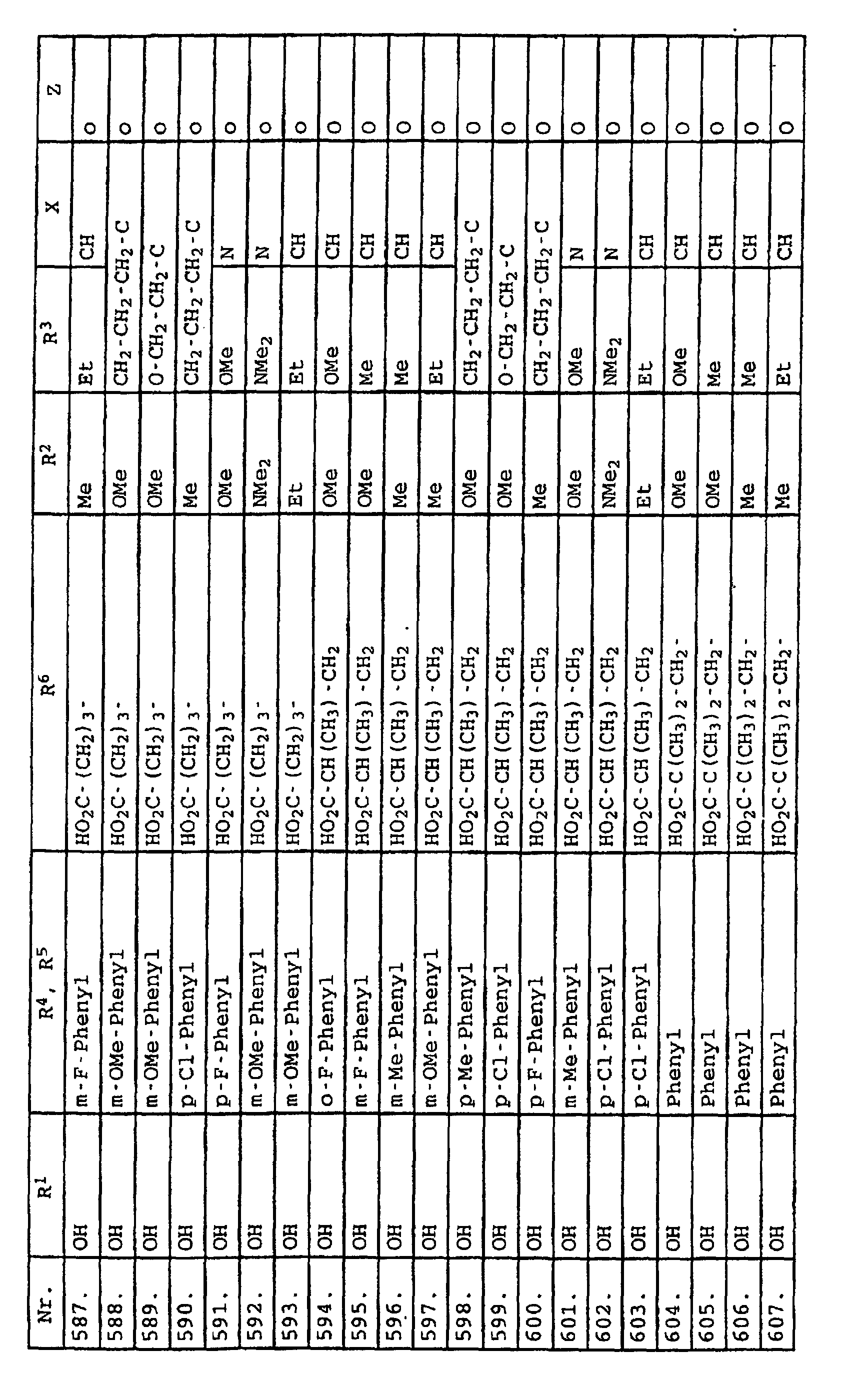

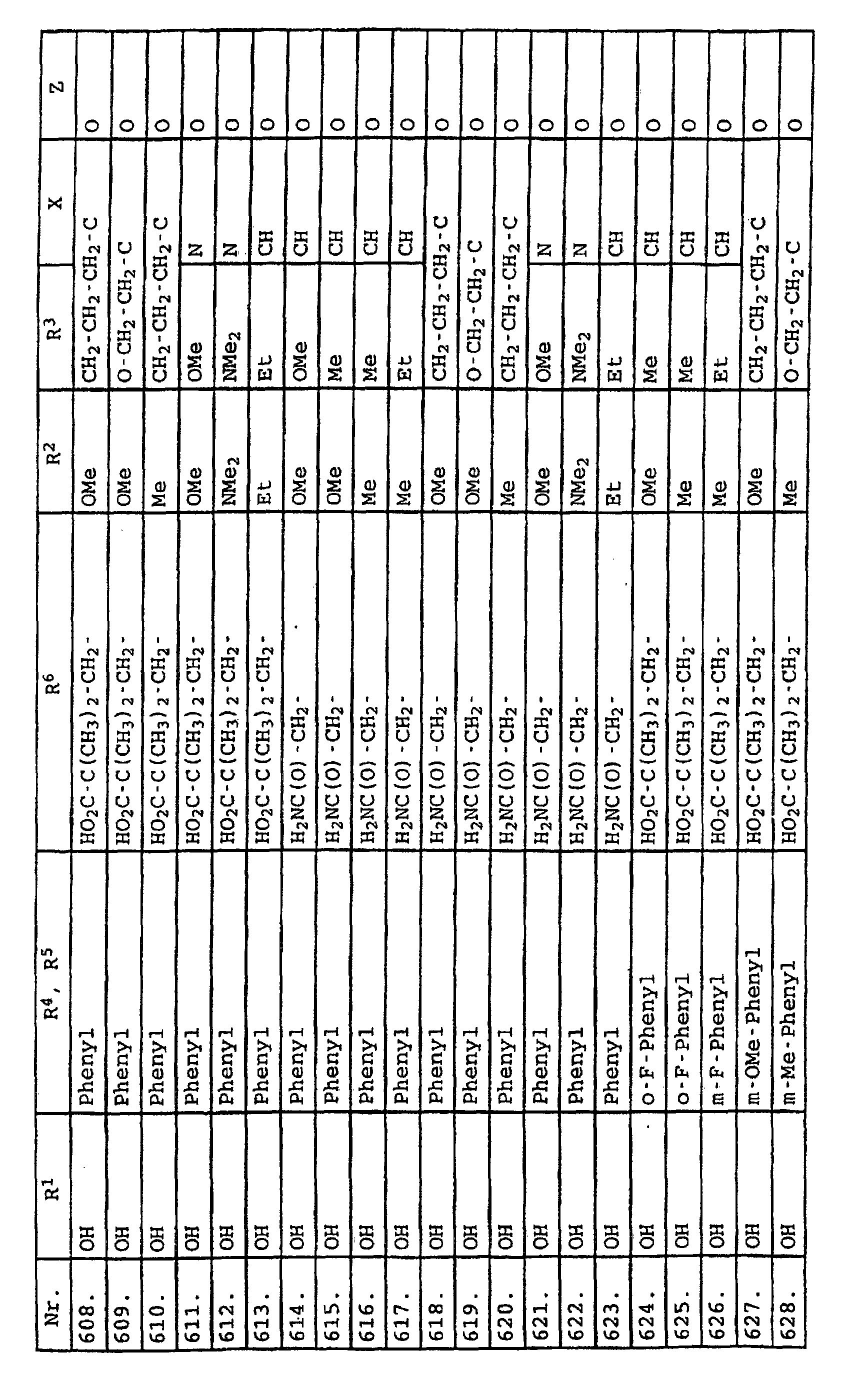

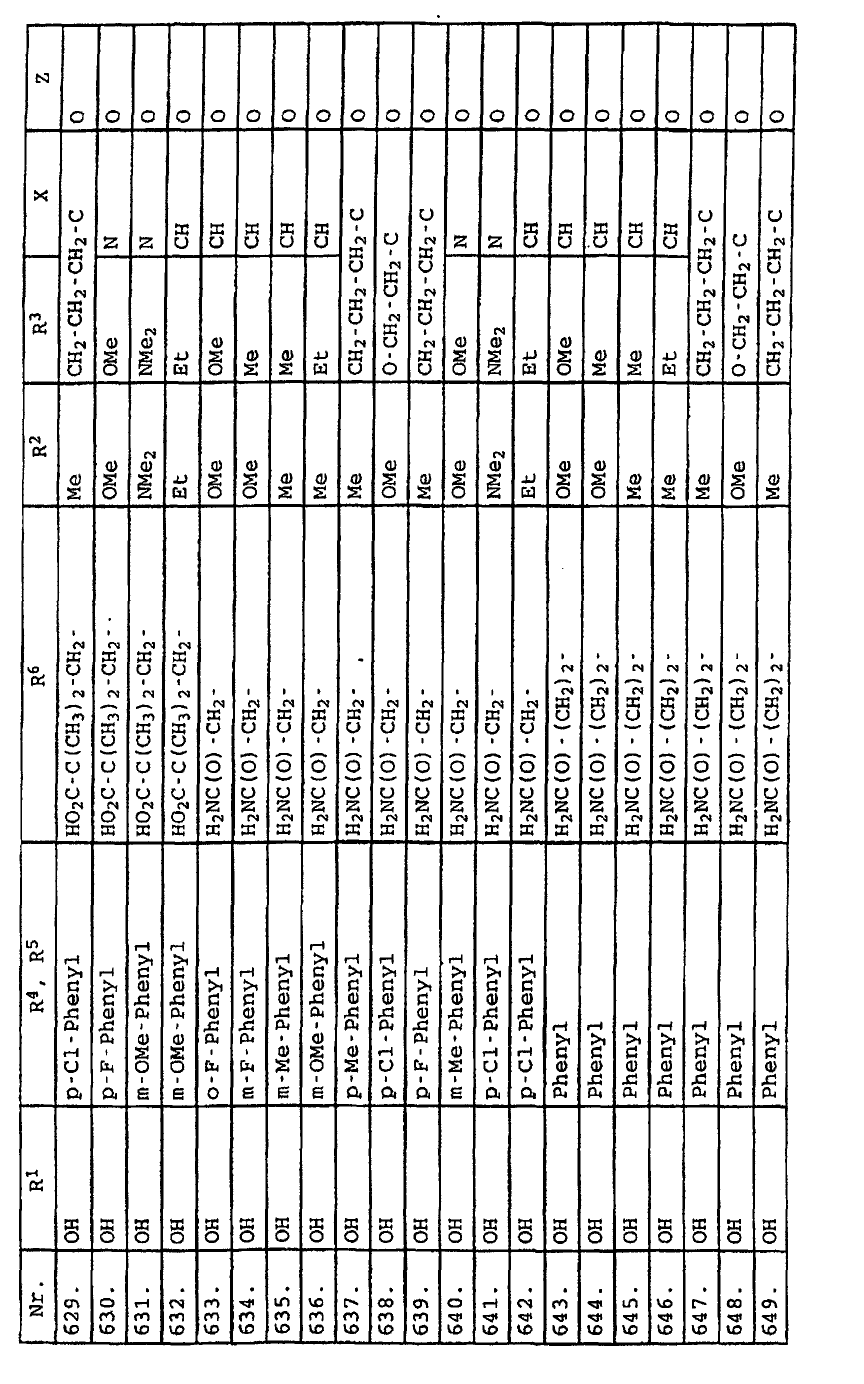

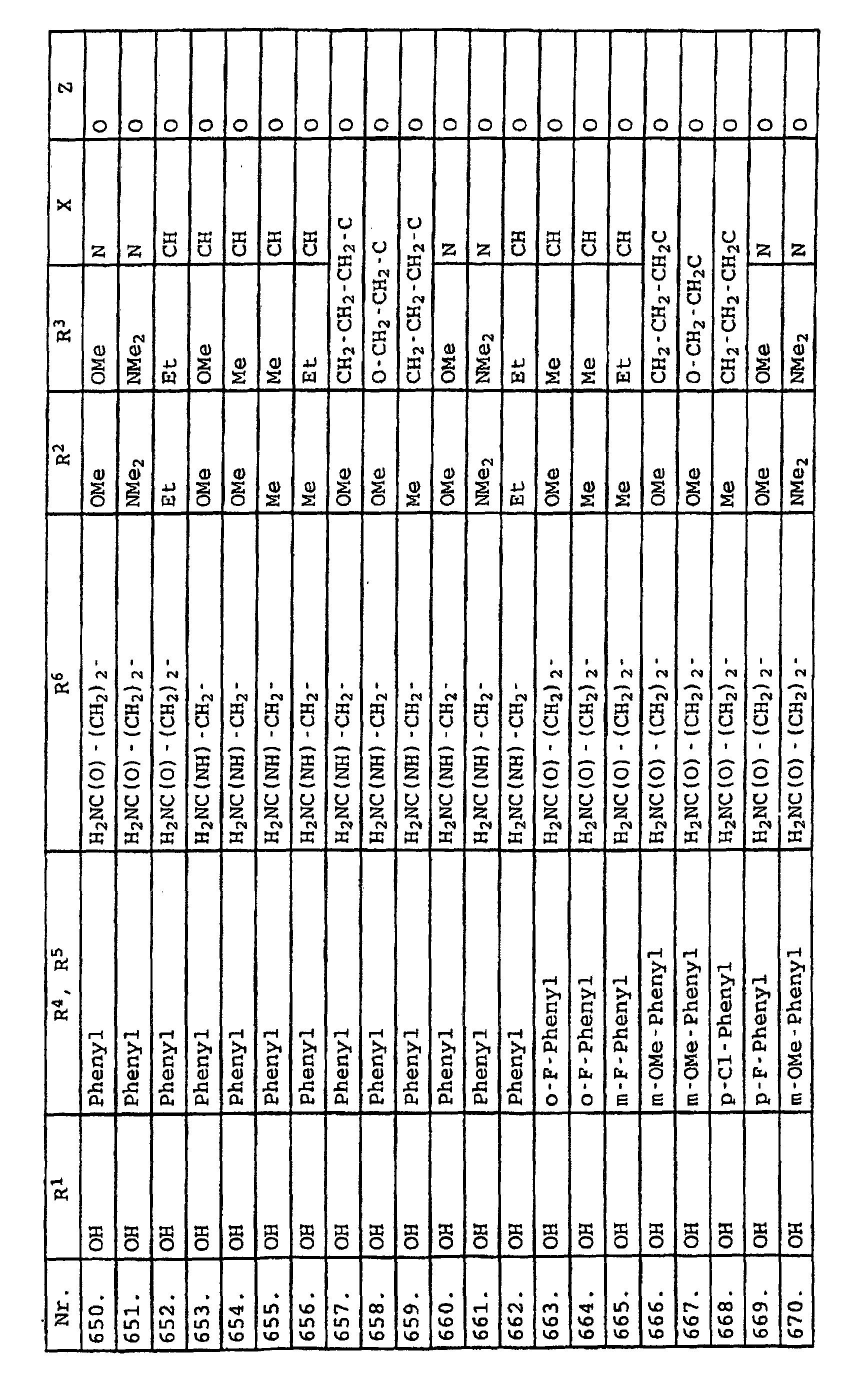

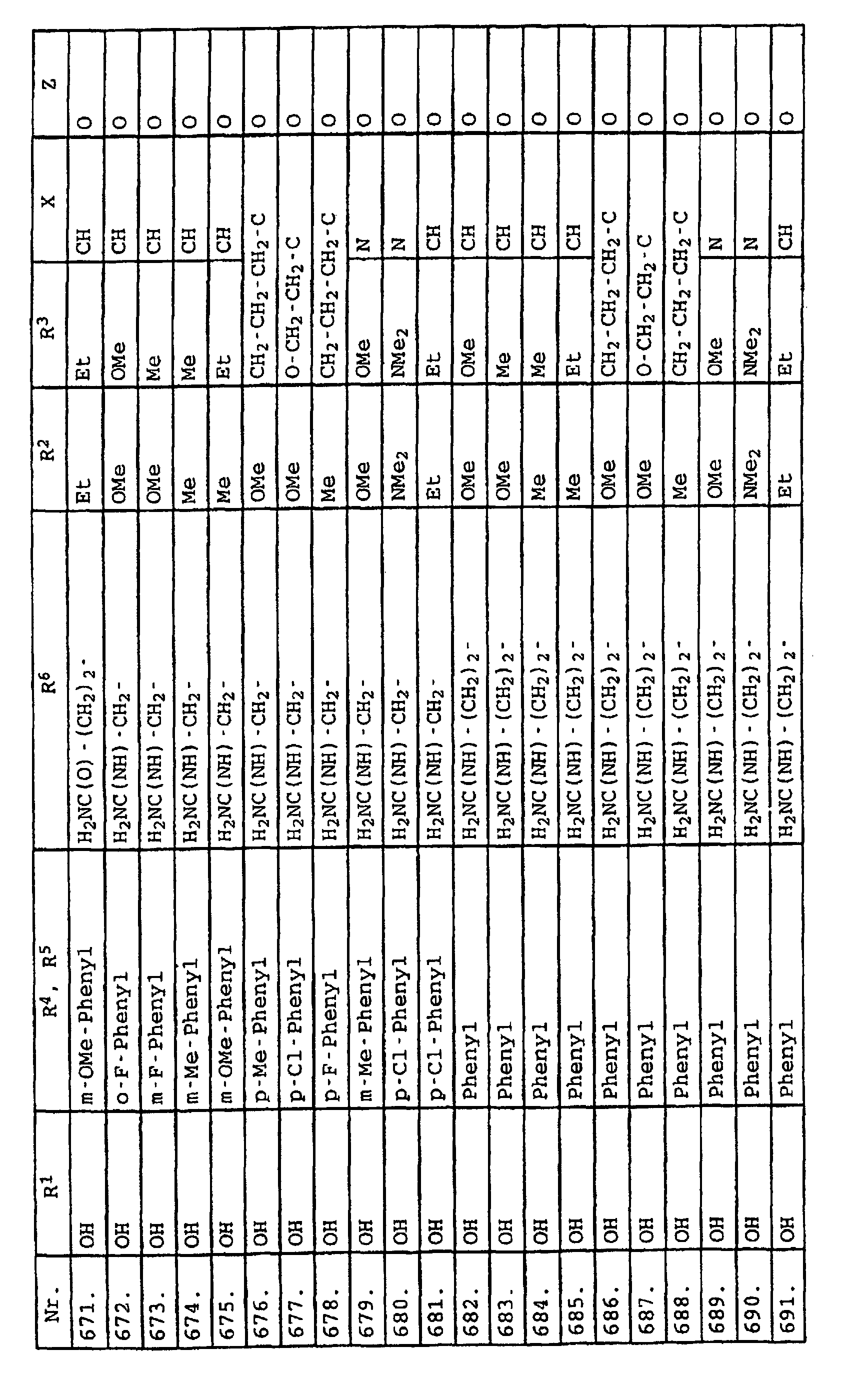

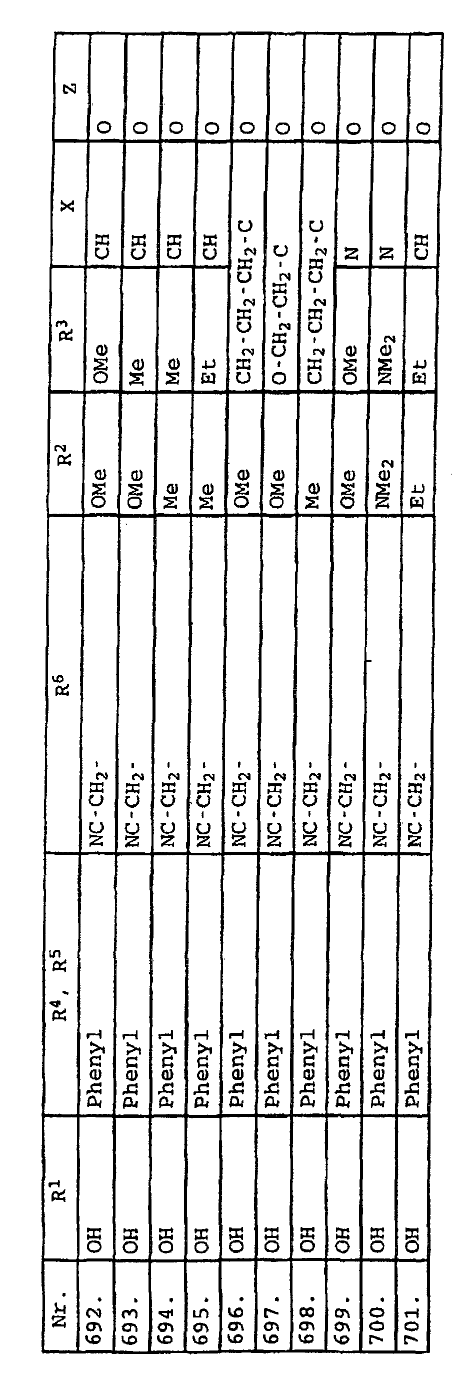

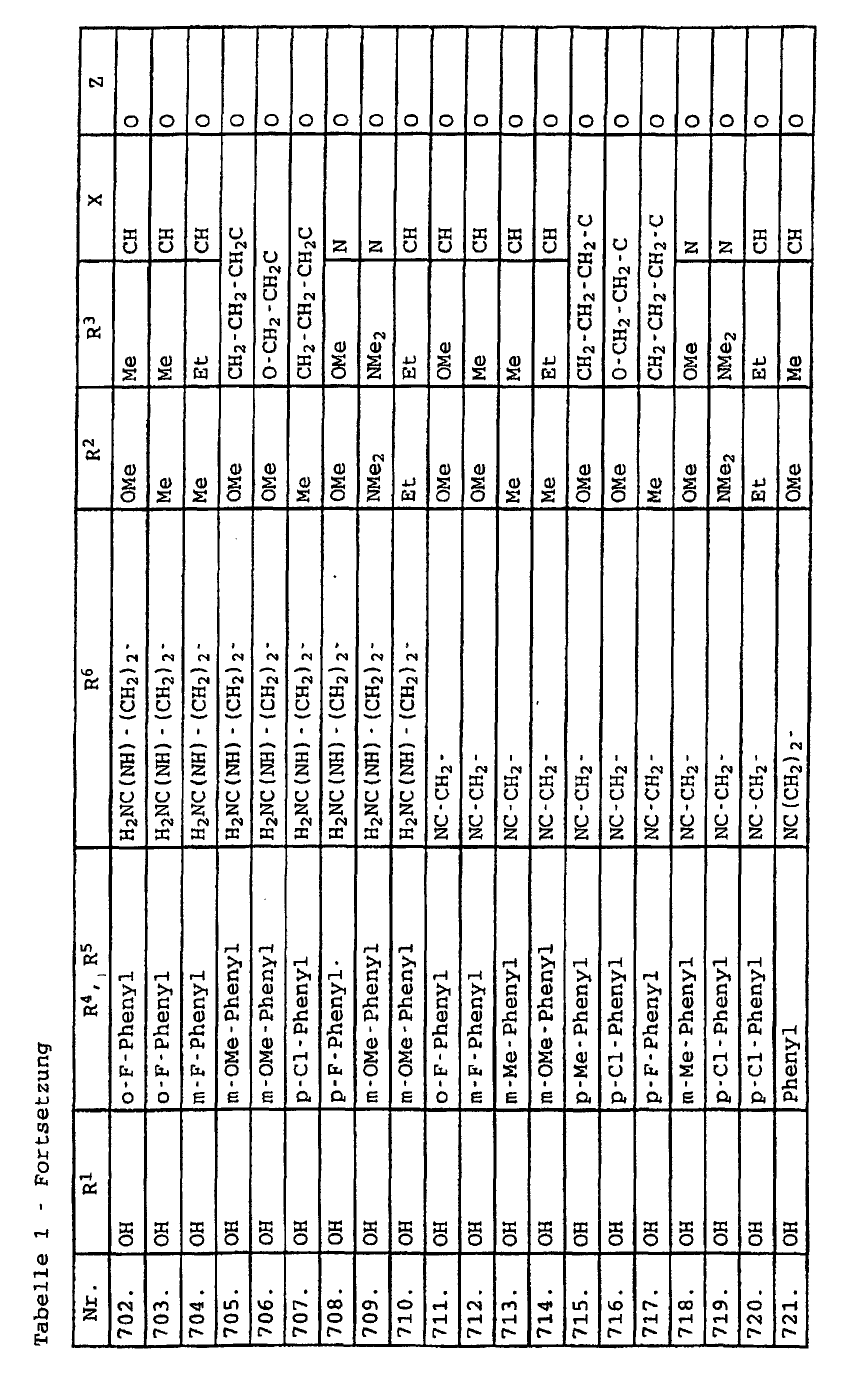

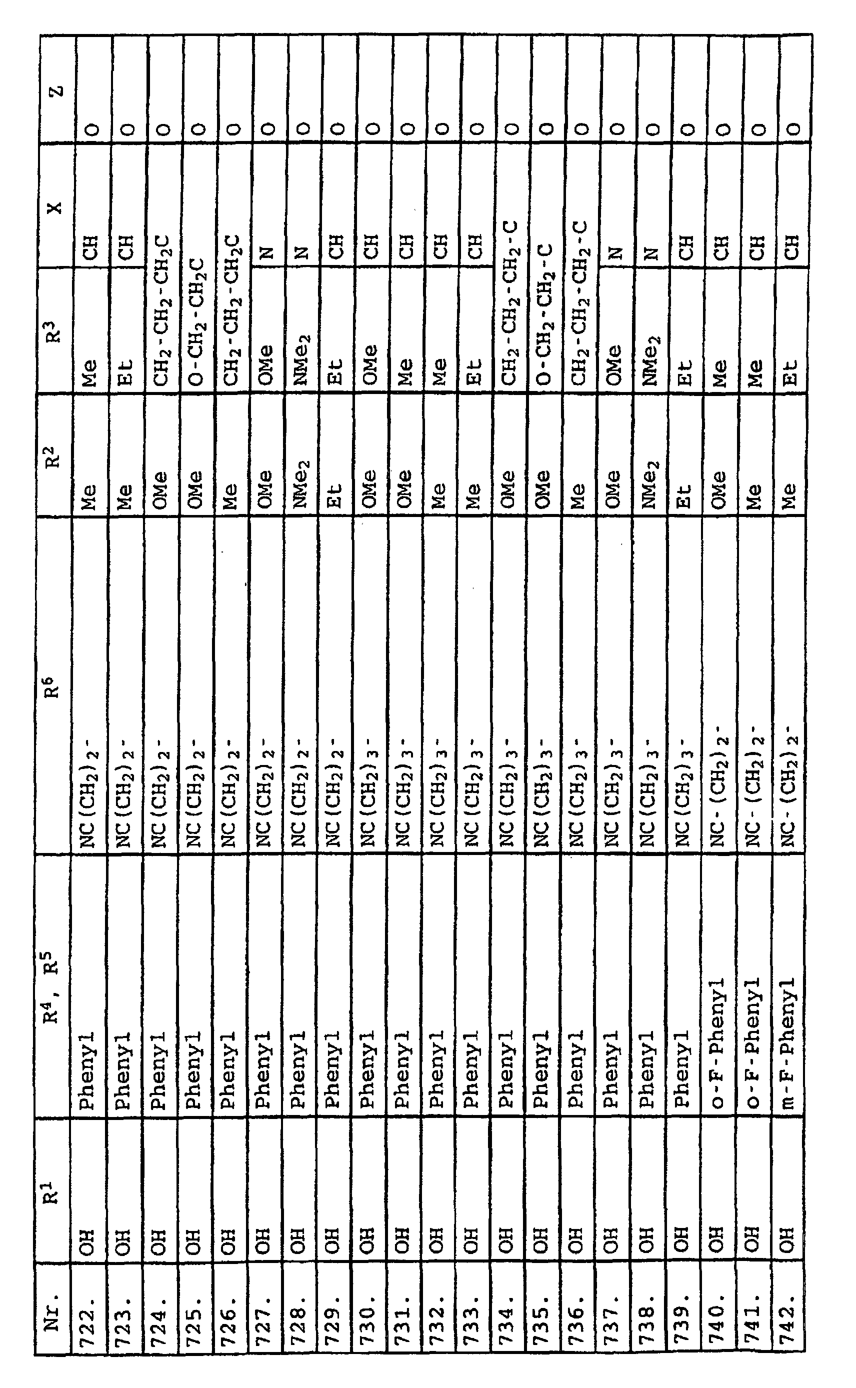

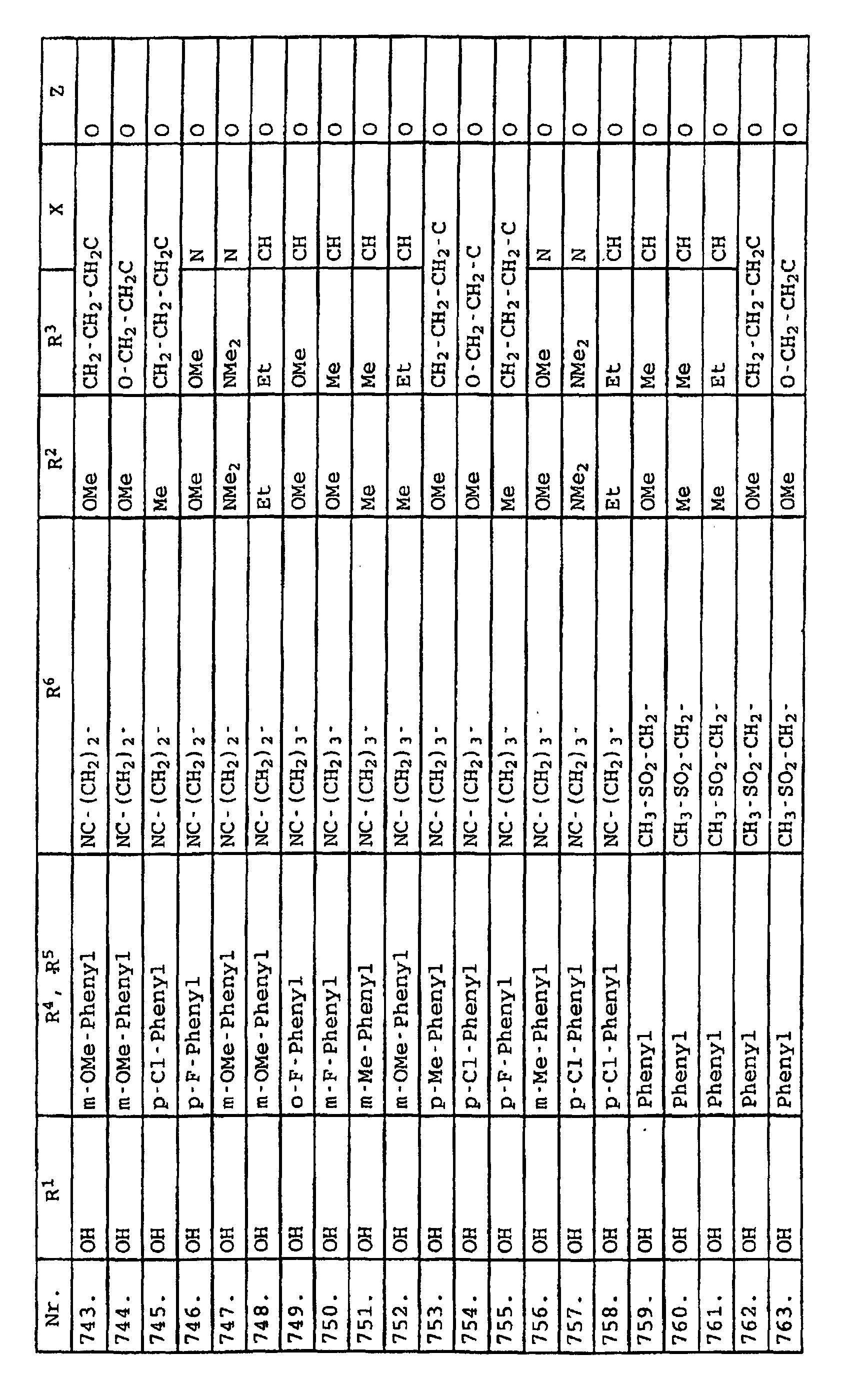

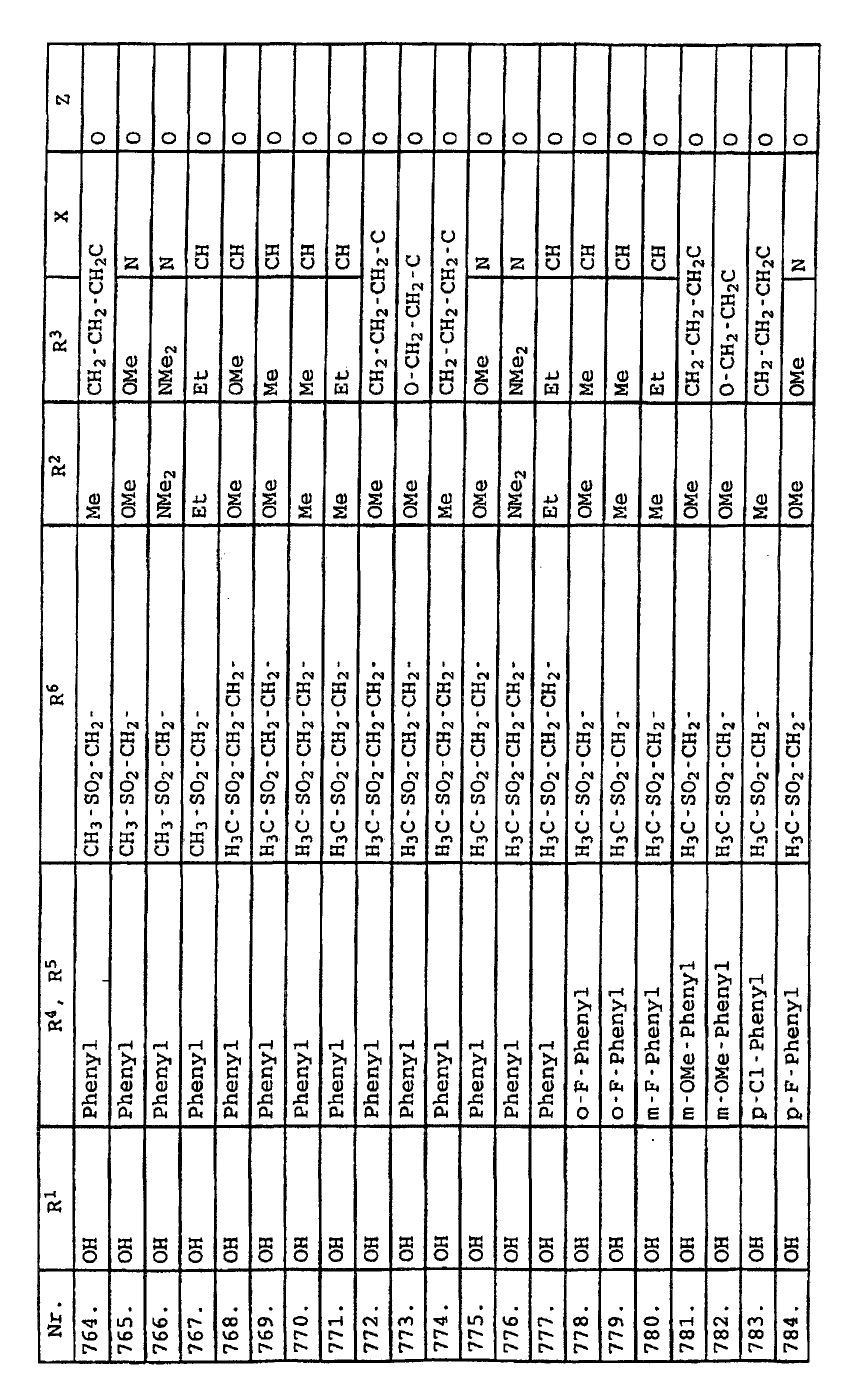

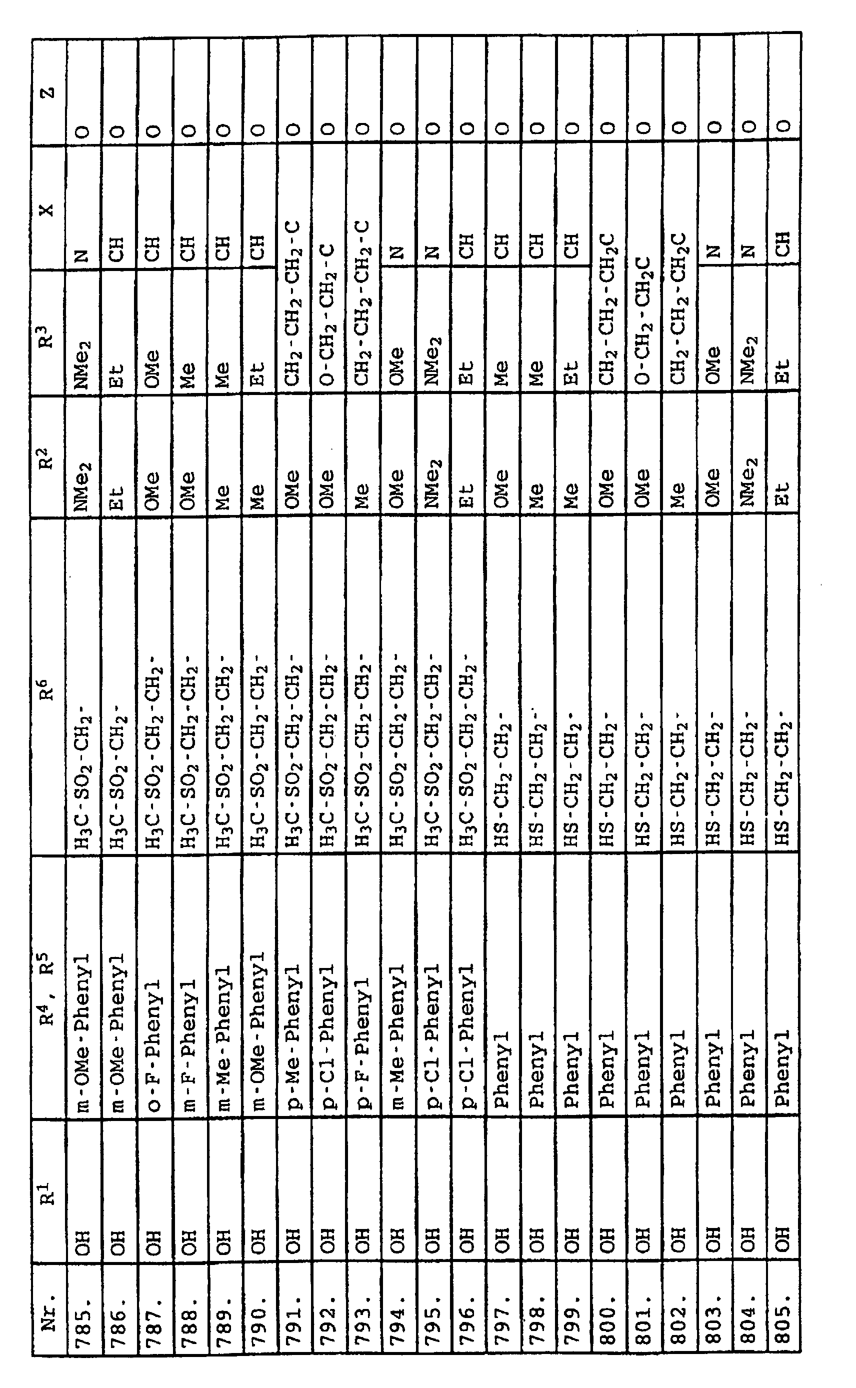

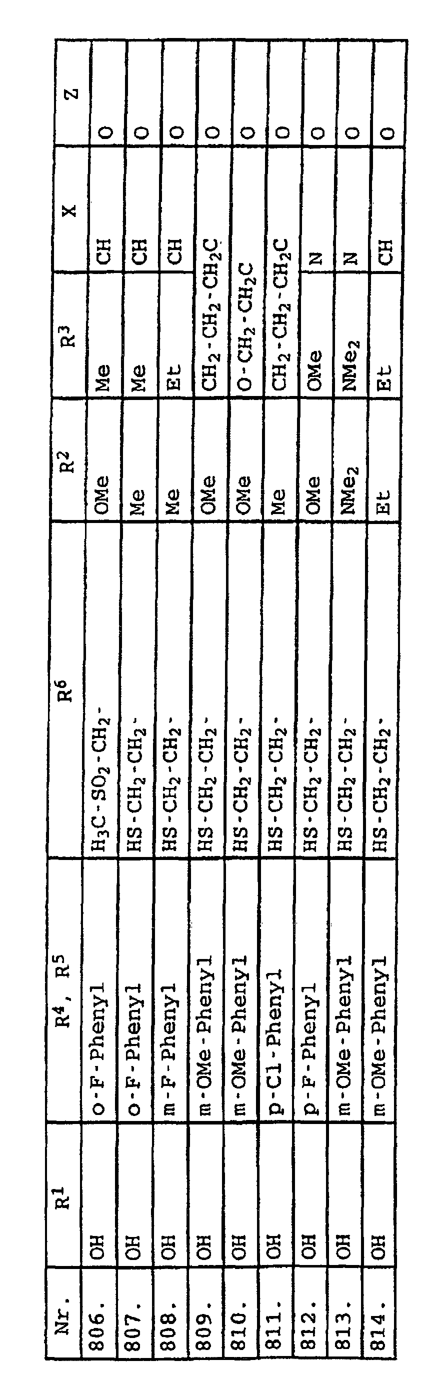

## Patentansprüche

1. Carbonsäurederivate der Formel I 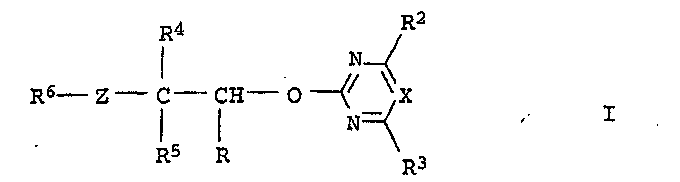 in der R ein Tetrazol, Nitril oder eine Gruppe 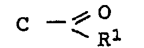 und die übrigen Substituenten folgende Bedeutung haben:
in der R¹ die folgende Bedeutung hat:
a) Wasserstoff;
b) eine Succinylimidoxygruppe;
c) ein über ein Stickstoffatom verknüpfter 5-gliedriger Heteroaromat wie Pyrrolyl, Pyrazolyl, Imidazolyl und Triazolyl, welcher ein bis zwei Halogenatome, insbesondere Fluor und Chlor und/oder ein bis zwei der folgenden Reste tragen kann:
C₁-C₄-Alkyl wie Methyl, Ethyl, 1-Propyl, 2-Propyl, 2-Methyl-2-propyl, 2-Methyl-1-propyl, 1-Butyl, 2-Butyl;
C₁-C₄-Halogenalkyl, insbesondere C₁-C₂-Halogenalkyl wie beispielsweise Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl;
C₁-C₄-Halogenalkoxy, insbesondere C₁-C₂-Halogenalkoxy wie Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;
C₁-C₄-Alkoxy wie Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy;
C₁-C₄-Alkylthio wie Methylthio, Ethylthio, Propylthio, 1-Methylethylthio, Butylthio, 1-Methylpropylthio, 2-Methylpropylthio, 1,1-Dimethylethylthio, insbesondere Methylthio und Ethylthio;
d) R¹ ferner ein Rest 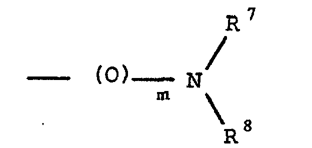 in dem m für 0 oder 1 steht und R⁷ und R⁸, die gleich oder unterschiedlich sein können, die folgende Bedeutung haben:
Wasserstoff
C₁-C₈-Alkyl,
C₃-C₆-Alkenyl,
C₃-C₆-Alkinyl,
C₃-C₈-Cycloalkyl, wobei diese Alkyl-, Cycloalkyl-, Alkenyl- und Alkinylgruppen jeweils ein bis fünf Halogenatome, insbesondere Fluor oder Chlor und/oder ein bis zwei der folgenden Gruppen tragen können:
C₁-C₄-Alkyl, C₁-C₄-Alkoxy, C₁-C₄-Alkylthio, C₁-C₄-Halogenalkoxy wie vorstehend genannt, C₃-C₆-Alkenyloxy, C₃-C₆-Alkenylthio, C₃-C₆-Alkinyloxy, C₃-C₆-Alkinylthio,
C₁-C₄-Alkylcarbonyl,
C₁-C₄-Alkoxycarbonyl,
C₃-C₆-Alkenylcarbonyl, C₃-C₆-Alkinylcarbonyl, C₃-C₆-Alkenyloxycarbonyl und C₃-C₆-Alkinyloxycarbonyl, Phenyl, gegebenenfalls ein- oder mehrfach, z.B. einbis dreifach substituiert durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio,
Di-C₁-C₄-Alkylamino,
R⁷ und R⁸ ferner Phenyl, das durch einen oder mehrere, der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio,
oder R⁷ und R⁸ bilden gemeinsam eine zu einem Ring geschlossene, C₄-C₇-Alkylenkette, die ein Heteroatom, ausgewählt aus der Gruppe Sauerstoff, Schwefel oder Stickstoff, enthalten kann,
e) R¹ ferner eine Gruppe 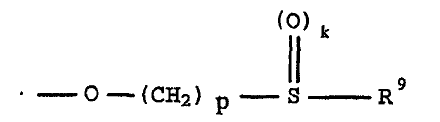 in der k die Werte 0, 1 und 2, p die Werte 1, 2, 3 und 4 annehmen und R⁹ für
C₁-C₄-Alkyl, C₁-C₄-Halogenalkyl, C₃-C₆-Alkenyl, C₃-C₆-Alkinyl oder gegebenenfalls substituiertes Phenyl steht,
f) R¹ ferner ein Rest OR¹⁰, worin R¹⁰ bedeutet:
Wasserstoff, das Kation eines Alkalimetalls wie Lithium, Natrium, Kalium oder das Kation eines Erdalkalimetalls wie Calcium, Magnesium und Barium oder ein organisches Ammoniumion;
C₃-C₈-Cycloalkyl, welches ein bis drei C₁-C₄-Alkylgruppen tragen kann;
C₁-C₈-Alkyl, welches ein bis fünf Halogenatome, und/oder einen der folgenden Reste tragen kann:
C₁-C₄-Alkoxy, C₁-C₄-Alkylthio, Cyano, C₁-C₄-Alkylcarbonyl, C₃-C₈-Cycloakyl, C₁-C₄-Alkoxycarbonyl, Phenyl, Phenoxy oder Phenylcarbonyl, wobei die aromatischen Reste ihrerseits jeweils ein bis fünf Halogenatome und/oder ein bis drei der folgenden Reste tragen können: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio;
eine C₁-C₈-Alkylgruppe, welche ein bis fünf Halogenatome, tragen kann und einen der folgenden Reste trägt: ein 5-gliedriger Heteroaromat, enthaltend ein bis drei Stickstoffatome, oder ein 5-gliedriger Heteroaromat enthaltend ein Stickstoffatom und ein Sauerstoff- oder Schwefelatom, welcher ein bis vier Halogenatome und/oder ein bis zwei der folgenden Reste tragen kann:
Nitro, Cyano, C₁-C₄-Alkyl, C₁-C₄-Halogenalkyl, C₁-C₄-Alkoxy, Phenyl, C₁-C₄-Halogenalkoxy und/oder C₁-C₄-Alkylthio.
eine C₂-C₆-Alkylgrupe, welche in der 2-Position einen der folgenden Reste trägt: C₁-C₄-Alkoxyimino, C₃-C₆-Alkinyloxyimino, C₃-C₆-Halogenalkenyloxyimino oder Benzyloxyimino;
eine C₃-C₆-Alkenyl- oder eine C₃-C₆-Alkinylgruppe, wobei diese Gruppen ihrerseits ein bis fünf Halogenatome tragen können;
R¹⁰ ferner ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio,
ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann;
R¹⁰ ferner ein Gruppe 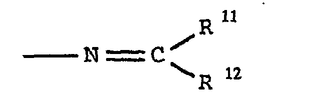 worin R¹¹ und R¹², die gleich oder verschieden sein können, bedeuten:
C₁-C₈-Alkyl, C₃-C₆-Alkenyl, C₃-C₆-Alkinyl, C₃-C₈-Cycloalkyl, wobei diese Reste einen C₁-C₄-Alkoxy, C₁-C₄-Alkylthio und/oder einen gegebenenfalls substituierten Phenylrest tragen können;
Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio,
oder R¹¹ und R¹² bilden gemeinsam eine C₃-C₁₂-Alkylenkette, welche ein bis drei C₁-C₄-Alkylgruppen tragen und ein Heteroatom aus der Gruppe Sauerstoff, Schwefel und Stickstoff enthalten kann.
g) R¹ ferner ein Rest 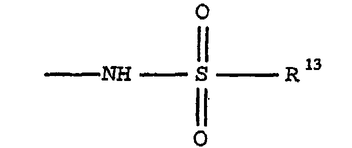 worin R¹³ bedeutet:
C₁-C₄-Alkyl, C₃-C₆-Alkenyl, C₃-C₆-Alkinyl, C₃-C₈-Cycloalkyl, wobei diese Reste einen C₁-C₄-Alkoxy-, C₁-C₄-Alkylthio- und/oder einen Phenylrest tragen können;
Phenyl, gegebenenfalls substituiert.
h) R¹ ein Rest 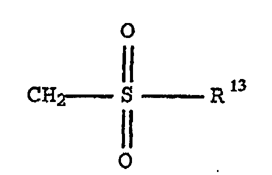 worin R¹³ die oben genannte Bedeutung hat.
R2 Halogen, C₁-C₄-Alkyl, C₁-C₄-Halogenalkyl, C₁-C₄-Alkoxy, C₁-C₄-Halogenalkoxy oder C₁-C₄-Alkylthio;
X Stickstoff oder CR¹⁴, worin R¹⁴ Wasserstoff oder C₁₋₅-Alkyl bedeutet oder CR¹⁴ zusammen mit CR³ einen 5- oder 6-gliedrigen Alkylen- oder Alkenylenring bildet, der durch eine oder zwei C₁₋₄-Alkylgruppen substituiert sein kann und worin jeweils eine Methylengruppe durch Sauerstoff, Schwefel, -NH oder -NC₁₋₄-Alkyl ersetzt sein kann;
R³ Halogen, C₁-C₄-Alkyl, C₁-C₄-Halogenalkyl, C₁-C₄-Alkoxy, C₁-C₄-Halogenalkoxy, -NH-O-C₁₋₄-Alkyl, C₁-C₄-Alkylthio oder CR³ ist mit CR¹⁴ wie oben angegeben zu einem 5- oder 6-gliedrigen Ring verknüpft;
R⁴ und R⁵ (die gleich oder verschieden sein können):
Phenyl oder Naphthyl, die durch einen oder mehrere der folgenden Reste substituiert sein können: Halogen, Nitro, Cyano, Hydroxy, C₁-C₄-Alkyl, C₁-C₄-Halogenalkyl, C₁-C₄-Alkoxy, C₁-C₄-Halogenalkoxy, Phenoxy, C₁-C₄-Alkylthio, Amino, C₁-C₄-Alkylamino oder C₁-C₄-Dialkylamino; oder
Phenyl oder Naphthyl, die orthoständig über eine direkte Bindung, eine Methylen-, Ethylen- oder Ethenylengruppe, ein Sauerstoff- oder Schwefelatom oder eine SO₂-, NH- oder N-Alkyl-Gruppe miteinander verbunden sind, oder C₃-C₇-Cycloalkyl;
R⁶ C₁-C₁₀-Alkyl, C₃-C₁₀-Alkenyl oder C₃-C₁₀-Alkinyl, wobei die Reste jeweils ein- oder mehrfach substituiert sind durch Hydroxy, Mercapto, Carboxy, 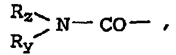 wobei R_{y} und R_{z} unabhängig voneinander Wasserstoff oder C₁-C₅-Alkyl bedeuten; Sulfonyl, Cyano, Guanidino, C₁-C₄-Alkoxy;
Z Schwefel oder Sauerstoff, mit der Maßgabe, dass : Carbonsäurederivate der Formel I ausgeschlossen sind, wenn der Rest R COOH, der Rest R² OMe, die Reste R⁴ und R⁵ Phenyl, und die Reste Y und Z O sind, und
(1) gleichzeitig der Rest R⁶ CH₂-CH₂-SO₂-CH(CH₃)₂, der Rest R³ OMe und der Rest X CH sind, oder
(2) gleichzeitig der Rest R⁶ CH₂-CH₂-SO₂-CH(CH₃)₂, der Rest R³ NH-OCH₃ und der Rest X CH sind, oder
(3) gleichzeitig der Rest R⁶ OH-CH₂-CH₂, der Rest R³ OMe und der Rest X CH sind, oder
(4) gleichzeitig der Rest R⁶ HOOC-(CH₂)₂-, der Rest R³ OMe und der Rest X CH sind, oder
(5) gleichzeitig der Rest R⁶ OH-CH₂-CH₂ und die Reste R³ und X zusammen einen Rest CH₂-CH₂-CH₂-C bilden sind.

2. Carbonsäurederivate nach Anspruch 1, **dadurch gekennzeichnet**, dap R=COOH bedeutet.

3. Carbonsäurederivatenach einem der obenstehenden Ansprüche, **dadurch gekennzeichnet, daß** mindestens einer der Reste R⁴ und R⁵ Phenyl bedeutet.

4. Carbonsäurederivate nach Anspruch 3, **dadurch gekennzeichnet, daß** R⁴ und R⁵ beide Phenyl bedeuten.

5. Carbonsäurederivate nach einem der obenstehenden Ansprüche, **dadurch gekennzeichnet, daß** R⁶=C₁-C₈-Alkyl, gegebenenfalls substituiert durch OH oder C₁-C₄-Alkoxy, und Z=O bedeuten.

6. Carbonsäurederivate nach einem der obenstehenden Ansprüche, **dadurch gekennzeichnet, daß** X=CH bedeutet.

7. Carbonsäurederivate nach einem der obenstehenden Ansprüche, **dadurch gekennzeichnet, daß** mindestens einer der Reste R², R³, C₁-C₄-Alkyl bedeutet.

8. Verwendung von Verbindungen gemäß Anspruch 1 bis 7 zur Herstellung von Medikamenten zur Behandlung von Hypertonie, pulmonalem Hochdruck, akutem und chronischem Nierenversagen, chronischer Herzinsuffizienz, zerebraler Ischämie, Restenose nach Angioplastie, Prostatakrebs.

9. Verwendung einer Kombination einer Verbindung gemäß Anspruch 1 bis 7 mit einem Inhibitor des Renin-Angiotensin-Systems (RAS).

## Claims

1. Carboxylic acid derivatives of the formula I 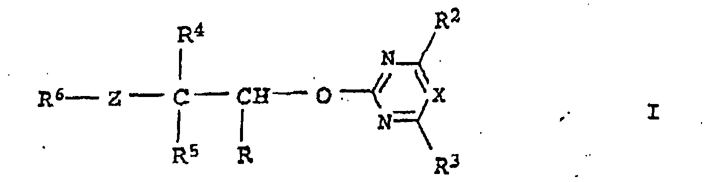 in which R is a tetrazole, nitrile or a group 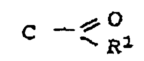 and the other substituents have the following meaning:
in which R¹ has the following meaning:
a) hydrogen;
b) a succinylimidoxy group;
c) a 5-membered heteroaromatic linked via a nitrogen atom, such as pyrrolyl, pyrazolyl, imidazolyl and triazolyl, which can carry one to two halogen atoms, in particular fluorine and chlorine, and/or one to two of the following radicals:
C₁-C₄-alkyl, such as methyl, ethyl, 1-propyl, 2-propyl, 2-methyl-2-propyl, 2-methyl-1-propyl, 1-butyl, 2-butyl;
C₁-C₄-halogenoalkyl, in particular C₁-C₂-halogenoalkyl, such as, for example, fluoromethyl, difluoromethyl, trifluoromethyl, chlorodifluoromethyl, dichlorofluoromethyl, trichloromethyl, 1-fluoroethyl, 2-fluoroethyl, 2,2-difluoroethyl, 2,2,2-trifluoroethyl, 2-chloro-2,2-difluoroethyl, 2,2-dichloro-2-fluoroethyl, 2,2,2-trichloroethyl and pentafluoroethyl;
C₁-C₄-halogenoalkoxy, in particular C₁-C₂-halogenoalkoxy, such as difluoromethoxy, trifluoromethoxy, chlorodifluoromethoxy, 1-fluoroethoxy, 2-fluoroethoxy, 2,2-difluoroethoxy, 1,1,2,2-tetrafluoroethoxy, 2,2,2-trifluoroethoxy, 2-chloro-1,1,2-trifluoroethoxy and pentafluoroethoxy, in particular trifluoromethoxy;
C₁-C₄-alkoxy, such as methoxy, ethoxy, propoxy, 1-methylethoxy, butoxy, 1-methylpropoxy, 2-methylpropoxy, 1,1-dimethylethoxy, in particular methoxy, ethoxy, 1-methylethoxy;
C₁-C₄-alkylthio, such as methylthio, ethylthio, propylthio, 1-methylethylthio, butylthio, 1-methylpropylthio, 2-methylpropylthio, 1,1-dimethylethylthio, in particular methylthio and ethylthio;
d) R¹ furthermore is a radical of the formula 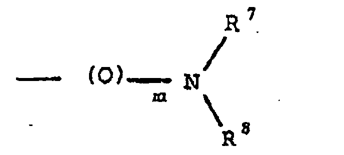 in which m represents 0 or 1 and R⁷ and R⁸, which can be identical or different, have the following meaning:
hydrogen
C₁-C₈-alkyl,
C₃-C₆-alkenyl,
C₃-C₆-alkinyl,
C₃-C₆-cycloalkyl, wherein these alkyl, cycloalkyl, alkenyl and alkinyl groups in each case can carry one to five halogen atoms, in particular fluorine or chlorine, and/or one to two of the following groups:
C₁-C₄-alkyl, C₁-C₄-alkoxy, C₁-C₄-alkylthio, C₁-C₄-halogenoalkoxy as mentioned above, C₃-C₆-alkenyloxy, C₃-C₆-alkenylthio, C₃-C₆-alkinyloxy, C₃-C₆-alkinylthio,
C₁-C₄-alkylcarbonyl,
C₁-C₄-alkoxycarbonyl,
C₃-C₆-alkenylcarbonyl, C₃-C₆-alkinylcarbonyl, C₃-C₆-alkenyloxycarbonyl and C₃-C₆-alkinyloxycarbonyl,
phenyl, optionally mono- or polysubstituted, e.g. mono- to trisubstituted by halogen, nitro, cyano, C₁-C₄-alkyl, C₁-C₄-halogenoalkyl, C₁-C₄-alkoxy, C₁-C₄-halogenoalkoxy or C₁-C₄-alkylthio,
di-C₁-C₄-alkylamino,
R⁷ and R⁸ furthermore are phenyl, which can be substituted by one or more of the following radicals: halogen, nitro, cyano, C₁-C₄-alkyl, C₁-C₄-halogenoalkyl, C₁-C₄-alkoxy, C₁-C₄-halogenoalkoxy or C₁-C₄-alkylthio,
or R⁷ and R⁸ together form a C₄-C₇-alkylene chain which is closed to form a ring and can contain a heteroatom chosen from the group consisting of oxygen, sulfur or nitrogen,
e) R¹ furthermore is a group 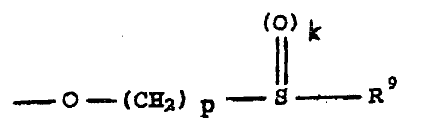 in which k assumes the values 0, 1 and 2, p assumes the values 1, 2, 3 and 4 and R⁹ represents
C₁-C₄-alkyl, C₁-C₄-halogenoalkyl, C₃-C₆-alkenyl, C₃-C₆-alkinyl or optionally substituted phenyl,
f) R¹ furthermore is a radical OR¹⁰, wherein R¹⁰ denotes:
hydrogen, the cation of an alkali metal, such as lithium, sodium, potassium, or the cation of an alkaline earth metal, such as calcium, magnesium and barium, or an organic ammonium ion;
C₃-C₈-cycloalkyl, which can carry one to three C₁-C₄-alkyl groups;
C₁-C₈-alkyl, which can carry one to five halogen atoms and/or one of the following radicals:
C₁-C₄-alkoxy, C₁-C₄-alkylthio, cyano, C₁-C₄-alkylcarbonyl, C₃-C₈-cycloalkyl, C₁-C₄-alkoxycarbonyl, phenyl, phenoxy or phenylcarbonyl, wherein the aromatic radicals in their turn in each case can carry one to five halogen atoms and/or one to three of the following radicals: nitro, cyano, C₁-C₄-alkyl, C₁-C₄-halogenoalkyl, C₁-C₄-alkoxy, C₁-C₄-halogenoalkoxy and/or C₁-C₄-alkylthio;
a C₁-C₈-alkyl group, which can carry one to five halogen atoms and carries one of the following radicals: a 5-membered heteroaromatic containing one to three nitrogen atoms, or a 5-membered heteroaromatic which contains a nitrogen atom and an oxygen or sulfur atom and can carry one to four halogen atoms and/or one to two of the following radicals:
nitro, cyano, C₁-C₄-alkyl, C₁-C₄-halogenoalkyl, C₁-C₄-alkoxy, phenyl, C₁-C₄-halogenoalkoxy and/or C₁-C₄-alkylthio;
a C₂-C₆-alkyl group, which carries one of the following radicals in the 2-position: C₁-C₄-alkoxyimino, C₃-C₆-alkinyloxyimino, C₃-C₆-halogenoalkenyloxyimino or benzyloxyimino;
a C₃-C₆-alkenyl or a C₃-C₆-alkinyl group, wherein these groups in their turn can carry one to five halogen atoms;
R¹⁰ furthermore is a phenyl radical, which can carry one to five halogen atoms and/or one to three of the following radicals: nitro, cyano, C₁-C₄-alkyl, C₁-C₄-halogenoalkyl, C₁-C₄-alkoxy, C₁-C₄-halogenoalkoxy and/or C₁-C₄-alkylthio,
a 5-membered heteroaromatic linked via a nitrogen atom, which contains one to three nitrogen atoms and can carry one to two halogen atoms and/or one to two of the following radicals;
R¹⁰ furthermore is a group 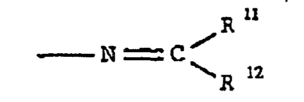 wherein R¹¹ and R¹², which can be identical or different, denote:
C₁-C₈-alkyl, C₃-C₆-alkenyl, C₃-C₆-alkinyl, C₃-C₈-cycloalkyl, wherein these radicals can carry a C₁-C₄-alkoxy, C₁-C₄-alkylthio and/or an optionally substituted phenyl radical;
phenyl, which can be substituted by one or more of the following radicals: halogen, nitro, cyano, C₁-C₄-alkyl, C₁-C₄-halogenoalkyl, C₁-C₄-alkoxy, C₁-C₄-halogenoalkoxy or C₁-C₄-alkylthio,
or R¹¹ and R¹² together form a C₃-C₁₂-alkylene chain, which can carry one to three C₁-C₄-alkyl groups and can contain a heteroatom from the group consisting of oxygen, sulfur and nitrogen;
g) R¹ furthermore is a radical 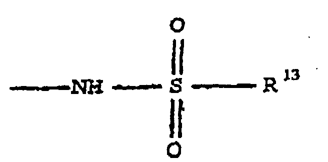 wherein R¹³ denotes:
C₁-C₄-alkyl, C₃-C₆-alkenyl, C₃-C₆-alkinyl, C₃-C₈-cycloalkyl, wherein these radicals can carry a C₁-C₄-alkoxy, C₁-C₄-alkylthio and/or a phenyl radical;
phenyl, optionally substituted;
h) R¹ is a radical 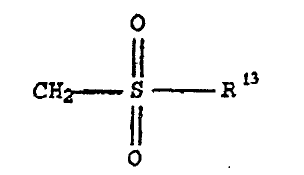 wherein R¹³ has the abovementioned meaning;
R² is halogen, C₁-C₄-alkyl, C₁-C₄-halogenoalkyl, C₁-C₄-alkoxy, C₁-C₄-halogenoalkoxy or C₁-C₄-alkylthio;
X is nitrogen or CR¹⁴, wherein R¹⁴ denotes hydrogen or C₁₋₅-alkyl, or CR¹⁴ together with CR³ forms a 5- or 6-membered alkylene or alkenylene ring, which can be substituted by one or two C₁₋₄-alkyl groups and wherein in each case a methylene group can be replaced by oxygen, sulfur, -NH or -NC₁₋₄-alkyl;
R³ is halogen, C₁-C₄-alkyl, C₁-C₄-halogenoalkyl, C₁-C₄-alkoxy, C₁-C₄-halogenoalkoxy, -NH-O-C₁₋₄-alkyl, C₁-C₄-alkylthio, or CR³ is linked with CR¹⁴ as mentioned above to form a 5- or 6-membered ring;
R⁴ and R⁵ (which can be identical or different) are:
phenyl or naphthyl, which can be substituted by one or more of the following radicals: halogen, nitro, cyano, hydroxyl, C₁-C₄-alkyl, C₁-C₄-halogenoalkyl, C₁-C₄-alkoxy, C₁-C₄-halogenoalkoxy, phenoxy, C₁-C₄-alkylthio, amino, C₁-C₄-alkylamino or C₁-C₄-dialkylamino; or
phenyl or naphthyl, which are bonded to one another in the ortho-position via a direct bond, a methylene, ethylene or ethenylene group, an oxygen or sulfur atom or an SO₂, NH or N-alkyl group, or C₃-C₇-cycloalkyl;
R⁶ is C₁-C₁₀-alkyl, C₃-C₁₀-alkenyl or C₃-C₁₀-alkinyl, wherein the radicals in each case are mono- or polysubstituted by hydroxyl, mercapto, carboxyl, 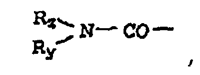 wherein R_{y} and R_{z} independently of one another denote hydrogen or C₁-C₅-alkyl; sulfonyl, cyano, guanidino, C₁-C₄-alkoxy;
Z is sulfur or oxygen, with the proviso that carboxylic acid derivatives of the formula I are excluded if the radical R is COOH, the radical R² is OMe, the radicals R⁴ and R⁵ are phenyl and the radicals Y and Z are O, and
(1) at the same time the radical R⁶ is CH₂-CH₂-SO₂-CH(CH₃)₂, the radical R³ is OMe and the radical X is CH, or
(2) at the same time the radical R⁶ is CH₂-CH₂-SO₂-CH(CH₃)₂, the radical R³ is NH-OCH₃ and the radical X is CH, or
(3) at the same time the radical R⁶ is OH-CH₂-CH₂, the radical R³ is OMe and the radical X is CH, or
(4) at the same time the radical R⁶ is HOOC-(CH₂)₂, the radical R³ is OMe and the radical X is CH, or
(5) at the same time the radical R⁶ is OH-CH₂-CH₂ and the radicals R³ and X together form a radical CH₂-CH₂-CH₂-C.

2. Carboxylic acid derivatives according to claim 1, **characterized in that** R denotes COOH.

3. Carboxylic acid derivatives according to one of the preceding claims, **characterized in that** at least one of the radicals R⁴ and R⁵ denotes phenyl.

4. Carboxylic acid derivatives according to claim 3, **characterized in that** R⁴ and R⁵ both denote phenyl.

5. Carboxylic acid derivatives according to one of the preceding claims, **characterized in that** R⁶ denotes C₁-C₈-alkyl, optionally substituted by OH or C₁-C₄-alkoxy, and Z denotes O.

6. Carboxylic acid derivatives according to one of the preceding claims, **characterized in that** X denotes CH.

7. Carboxylic acid derivatives according to one of the preceding claims, **characterized in that** at least one of the radicals R², R³ denotes C₁-C₄-alkyl.

8. Use of compounds according to claim 1 to 7 for the preparation of medicaments for treatment of hypertension, pulmonary hypertension, acute and chronic renal failure, chronic cardiac insufficiency, cerebral ischaemia, restenosis following angioplasty, prostate cancer.

9. Use of a combination of a compound according to claim 1 to 7 with an inhibitor of the renin-angiotensin system (RAS).

## Revendications

1. Dérivés d'acides carboxyliques de formule I 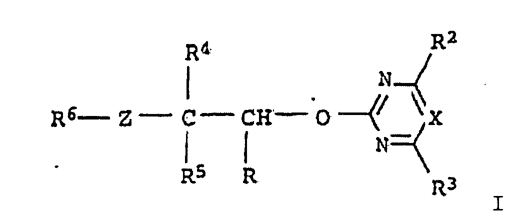 dans laquelle R est un tétrazole, un nitrile, ou un groupe 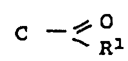 et les autres substituants ont la signification suivante :
dans laquelle R¹ a la signification suivante :
a) un hydrogène ;
b) un groupe succinylimidoxy ;
c) un groupe hétéroaromatique à 5 chaînons lié au moyen d'un atome d'azote, comme un groupe pyrrolyle, un pyrazolyle, un imidazolyle et un triazolyle, lequel peut porter un à deux atomes d'halogène, en particulier le fluor et le chlore et/ou un à deux des radicaux suivants :
un groupe alkyle en C₁ à C₄, comme un méthyle, un éthyle, un 1-propyle, un 2-propyle, un 2-méthyl-2-propyle, un 2-méthyl-1-propyle, un 1-butyle, un 2-butyle,
un groupe alkyle halogéné en C₁ à C₄, en particulier un groupe alkyle halogéné en C₁ à C₂, comme par exemple un fluorométhyle, un difluorométhyle, un trifluorométhyle, un chlorodifluorométhyle, un dichlorofluorométhyle, un trichlorométhyle, un 1-fluoroéthyle, un 2-fluoroéthyle, un 2,2-difluoroéthyle, un 2,2,2-trifluoroéthyle, un 2-chloro-2,2-difluoroéthyle, un 2,2-dichloro-2-fluoroéthyle, un 2,2,2-trichloroéthyle et un pentafluoroéthyle ;
un groupe alcoxy halogéné en C₁ à C₄, en particulier un groupe alcoxy halogéné en C₁ à C₂ comme un difluorométhoxy, un trifluorométhoxy, un chlorodifluorométhoxy, un 1-fluoroéthoxy, un 2-fluoroéthoxy, un 2,2-difluoroéthoxy, un 1,1,2,2-tétrafluoroéthoxy, un 2,2,2-trifluoroéthoxy, un 2-chloro-1,1,2-trifluoroéthoxy et un pentafluoroéthoxy, en particulier un trifluorométhoxy ;
un groupe alcoxy en C₁ à C₄ comme un méthoxy, un éthoxy, un propoxy, un 1-méthyléthoxy, un butoxy, un 1-méthylpropoxy, un 2-méthylpropoxy, un 1,1-diméthyléthoxy, en particulier un méthoxy, un éthoxy, un 1-méthyléthoxy ;
un groupe alkylthio en C₁ à C₄ comme un méthylthio, un éthylthio, un propylthio, un 1-méthyléthylthio, un butylthio, un 1-méthylpropylthio, un 2-méthylpropylthio, un 1,1-diméthyléthylthio, en particulier un méthylthio et un éthylthio ;
d) R¹ pouvant être en outre un radical 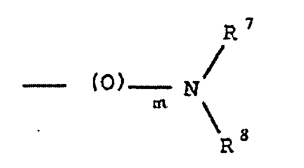 dans lequel m représente 0 ou 1 et R⁷ et R⁸, qui peuvent être identiques ou différents, ont la signification suivante :
un hydrogène,
un groupe alkyle en C₁ à C₈,
un groupe alcényle en C₃ à C₆,
un groupe alcinyle en C₃ à C₆,
un groupe cycloalkyle en C₃ à C₈,
ces groupes alkyles, cycloalkyles, alcényles et alcinyles pouvant porter respectivement un à cinq atomes d'halogènes, en particulier le fluor ou le chlore et/ou un à deux des groupes suivants :
un groupe alkyle en C₁ à C₄, un groupe alcoxy en C₁ à C₄, un groupe alkylthio en C₁ à C₄, un groupe alcoxy halogéné en C₁ à C₄ comme dénommé précédemment, un groupe alcényloxy en C₃ à C₆, un groupe alcénylthio en C₃ à C₆, un groupe alcinyloxy en C₃ à C₆, un groupe alcinylthio en C₃ à C₆,
un groupe alkylcarbonyle en C₁ à C₄,
un groupe alcoxycarbonyle en C₁ à C₄,
un groupe alcénylcarbonyle en C₃ à C₆, un groupe alcinylcarbonyle en C₃ à C₆,
un groupe alcényloxycarbonyle en C₃ à C₆ et un groupe alcinyloxycarbonyle en C₃ à C₆,
un phényle, le cas échéant substitué une ou plusieurs fois, par exemple une à trois fois par un halogène, un groupe nitro, cyano, un groupe alkyle en C₁ à C₄, un groupe alkyle halogéné en C₁ à C₄, un groupe alcoxy en C₁ à C₄, un groupe alcoxy halogéné en C₁ à C₄ ou un groupe alkylthio en C₁ à C₄,
un groupe dialkylamino en C₁ à C₄,
R⁷ et R⁸ signifiant en outre un phényle, qui peut être substitué par un ou plusieurs des radicaux suivants : un halogène, un groupe nitro, cyano, un groupe alkyle en C₁ à C₄, un groupe alkyle halogéné en C₁ à C₄, un groupe alcoxy en C₁ à C₄, un groupe alcoxy halogéné en C₁ à C₄ ou un groupe alkylthio en C₁ à C₄,
ou R⁷ et R⁸ forment ensemble une chaîne alkylène en C₄ à C₇ fermée en un cycle qui peut contenir un hétéroatome choisi parmi le groupe constitué de l'oxygène, du soufre ou de l'azote,
e) R¹ pouvant signifier en outre un groupe 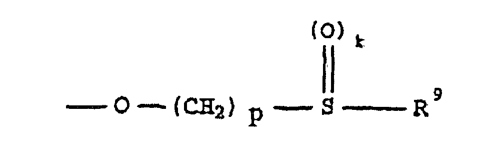 dans lequel k prend les valeurs 0, 1 et 2, p les valeurs 1, 2, 3 et 4 et R⁹ représente
un groupe alkyle en C₁ à C₄, un groupe alkyle halogéné en C₁ à C₄, un groupe alcényle en C₃ à C₆, un groupe alcinyle en C₃ à C₆ ou phényle substitué le cas échéant
f) R¹ pouvant signifier en outre un radical OR¹⁰, dans lequel R¹⁰ signifie :
un hydrogène, le cation d'un métal alcalin comme le lithium, le sodium, le potassium ou le cation d'un métal alcalinoterreux comme le calcium, le magnésium et le baryum ou un ion ammonium organique ;
un groupe cycloalkyle en C₃ à C₈, lequel peut porter un à trois groupes alkyles en C₁ à C₄,
un groupe alkyle en C₁ à C₈, lequel peut porter un à cinq atomes d'halogènes et/ou un des radicaux suivants :
un groupe alcoxy en C₁ à C₄, un groupe alkylthio en C₁ à C₄, un groupe cyano, un groupe alkylcarbonyle en C₁ à C₄, un groupe cycloalkyle en C₃ à C₈, un groupe alcoxycarbonyle en C₁ à C₄, un phényle, un groupe phénoxy ou phénylcarbonyle, les radicaux aromatiques pouvant porter de leur côté respectivement un à cinq atomes d'halogènes et/ou un à trois des radicaux suivants : un groupe nitro, cyano, un groupe alkyle en C₁ à C₄, un groupe alkyle halogéné en C₁ à C₄, un groupe alcoxy en C₁ à C₄, un groupe alcoxy halogéné en C₁ à C₄ et/ou un groupe alkylthio en C₁ à C₄,
un groupe alkyle en C₁ à C₈, lequel peut porter un à cinq atomes d'halogènes et porte un des radicaux suivants : un groupe hétéroaromatique à 5 chaînons contenant un à trois atomes d'azote, ou un groupe hétéroaromatique à 5 chaînons contenant un atome d'azote et un atome d'oxygène ou de soufre, lequel groupe peut porter un à quatre atomes d'halogènes et/ou un à deux des radicaux suivants :
un groupe nitro, cyano, un groupe alkyle en C₁ à C₄, un groupe alkyle halogéné en C₁ à C₄, un groupe alcoxy en C₁ à C₄, un phényle, un groupe alcoxy halogéné en C₁ à C₄ et/ou un groupe alkylthio en C₁ à C₄,
un groupe alkyle en C₂ à C₆, lequel porte en position 2 un des radicaux suivants : un groupe alcoxyimino en C₁ à C₄, un groupe alcinyloxyimino en C₃ à C₆, un groupe alcényloxyimino halogéné en C₃ à C₆ ou un groupe benzyloxyimino ;
un groupe alcényle ou alcinyle en C₃ à C₆, ces groupes pouvant de leur côté porter un à cinq atomes d'halogènes ;
R¹⁰ pouvant signifier en outre un radical phényle, lequel peut porter un à cinq atomes d'halogènes et/ou un à trois des radicaux suivants : un groupe nitro, cyano, un groupe alkyle en C₁ à C₄, un groupe alkyle halogéné en C₁ à C₄, un groupe alcoxy en C₁ à C₄, un groupe alcoxy halogéné en C₁ à C₄ et/ou un groupe alkylthio en C₁ à C₄,
un groupe hétéroaromatique à 5 chaînons lié au moyen d'un atome d'azote, contenant un à trois atomes d'azote, lequel peut porter un à deux atomes d'halogènes et/ou un à deux des radicaux suivants :
R¹⁰ pouvant signifier en outre un groupe 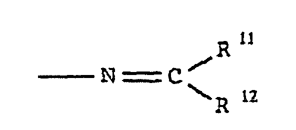 dans lequel R¹¹ et R¹², qui peuvent être identiques ou différents, signifient :
un groupe alkyle en C₁ à C₈, un groupe alcényle en C₃ à C₆, un groupe alcinyle en C₃ à C₆, un groupe cycloalkyle en C₃ à C₈, ces radicaux pouvant porter un groupe alcoxy en C₁ à C₄, un groupe alkylthio en C₁ à C₄ et/ou un radical phényle substitué le cas échéant ;
un phényle qui peut être substitué par un ou plusieurs des radicaux suivants : un halogène, un groupe nitro, cyano, un groupe alkyle en C₁ à C₄, un groupe alkyle halogéné en C₁ à C₄, un groupe alcoxy en C₁ à C₄, un groupe alcoxy halogéné en C₁ à C₄ ou un groupe alkylthio en C₁ à C₄,
ou R¹¹ et R¹² forment ensemble une chaîne alkylène en C₃ à C₁₂, laquelle peut porter un à trois groupes alkyles en C₁ à C₄ et contenir un hétéroatome choisi dans le groupe consistant en l'oxygène, le soufre et l'azote
g) R¹ pouvant signifier en outre un radical 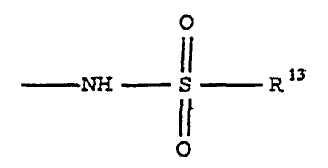 dans lequel R¹³ signifie :
un groupe alkyle en C₁ à C₄, un groupe alcényle en C₃ à C₆, un groupe alcinyle en C₃ à C₆, un groupe cycloalkyle en C₃ à C₈, ces radicaux pouvant porter un groupe alcoxy en C₁ à C₄, un groupe alkylthio en C₁ à C₄ et/ou un radical phényle ;
un phényle, substitué le cas échéant.
h) R¹ pouvant signifier un radical 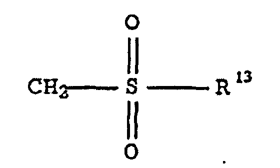 dans lequel R¹³ a la signification dénommée ci-dessus;
R² signifie un halogène, un groupe alkyle en C₁ à C₄, un groupe alkyle halogéné en C₁ à C₄, un groupe alcoxy en C₁ à C₄, un groupe alcoxy halogéné en C₁ à C₄ ou un groupe alkylthio en C₁ à C₄,
X signifie l'azote ou un groupe CR¹⁴, dans lequel R¹⁴ signifie l'hydrogène ou un alkyle en C₁₋₅ ou CR¹⁴ forme conjointement avec CR³ un cycle alkylène ou alcénylène à 5 ou 6 chaînons qui peut être substitué par un ou deux groupes alkyles en C₁₋₄ et dans lequel respectivement un groupe méthylène peut être remplacé par l'oxygène, le soufre, un groupe -NH ou un groupe N-alkyle en C₁₋₄ ;
R³ signifie un halogène, un groupe alkyle en C₁ à C₄, un groupe alkyle halogéné en C₁ à C₄, un groupe alcoxy en C₁ à C₄, un groupe alcoxy halogéné en C₁ à C₄, un groupe -NH-O-C₁₋₄-alkyle, un groupe alkylthio en C₁ à C₄ ou un groupe CR³ combiné avec CR¹⁴ comme indiqué ci-dessus en un cycle à 5 ou 6 chaînons ;
R⁴ et R⁵ (qui peuvent être identiques ou différents) sont :
un groupe phényle ou naphtyle, qui peuvent être substitués par un ou plusieurs des radicaux suivants : un halogène, un groupe nitro, cyano, hydroxy, un groupe alkyle en C₁ à C₄, un groupe alkyle halogéné en C₁ à C₄, un groupe alcoxy en C₁ à C₄, un groupe alcoxy halogéné en C₁ à C₄, un groupe phénoxy, un groupe alkylthio en C₁ à C₄, un groupe amino, un groupe alkylamino en C₁ à C₄ ou dialkylamino en C₁ à C₄ ; ou
des groupes phényle ou naphtyle, qui sont liés l'un à l'autre en position ortho au moyen d'une liaison directe, d'un groupe méthylène, éthylène ou éthénylène, d'un atome d'oxygène ou de soufre ou d'un groupe SO₂-, NH- ou N-alkyle, ou un groupe cycloalkyle en C₃ à C₇,
R⁶ signifie un groupe alkyle en C₁ à C₁₀, un groupe alcényle C₃ à C₁₀ ou alcinyle en C₃ à C₁₀, les radicaux étant respectivement substitués une ou plusieurs fois par un groupe hydroxy, mercapto, carboxy, 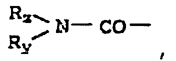 R_{y} et R_{z} signifiant indépendamment l'un de l'autre un hydrogène ou un groupe alkyle en C₁ à C₅ ; un groupe sulfonyle, cyano, guanidino, un groupe alcoxy en C₁ à C₄,
Z signifie le soufre ou l'oxygène, à condition que les dérivés d'acides carboxyliques de formule I soient exclus quand le radical R est COOH, le radical R² est Ome, les radicaux R⁴ et R⁵ sont un phényle, et les radicaux Y et Z sont O, et
(1) les radicaux sont en même temps : R⁶ = CH₂-CH₂-SO₂-CH(CH₃)₂, R³ = OMe et X = CH, ou
(2) les radicaux sont en même temps : R⁶ = CH₂-CH₂-SO₂-CH(CH₃)₂, R³ = NH-OCH₃ et X = CH, ou
(3) les radicaux sont en même temps : R⁶ = OH-CH₂-CH₂, R³ = OMe et X = CH, ou
(4) les radicaux sont en même temps : R⁶ = HOOC-(CH₂)₂⁻, R³ = OMe et X = CH, ou
(5) les radicaux sont en même temps : R6 = OH-CH₂-CH₂ et R³ et X forment ensemble un radical CH₂-CH₂-CH₂-C.

2. Dérivés d'acides carboxyliques selon la revendication 1, **caractérisés en ce que** R signifie COOH.

3. Dérivés d'acides carboxyliques selon l'une quelconque des revendications précédentes, **caractérisés en ce qu'**au moins un des radicaux R⁴ et R⁵ signifie le phényle.

4. Dérivés d'acides carboxyliques selon la revendication 3, **caractérisés en ce que** R⁴ et R⁵ signifient tous les deux le phényle.

5. Dérivés d'acides carboxyliques selon l'une quelconque des revendications précédentes, **caractérisés en ce que** R⁶ signifie un groupe alkyle en C₁ à C₈, le cas échéant substitué par un OH ou un groupe alcoxy en C₁ à C₄, et **en ce que** Z signifie O.

6. Dérivés d'acides carboxyliques selon l'une quelconque des revendications précédentes, **caractérisés en ce que** X signifie CH.

7. Dérivés d'acides carboxyliques selon l'une quelconque des revendications précédentes, **caractérisés en ce qu'**au moins un des radicaux R², R³ signifie un groupe alkyle en C₁ à C4.

8. Utilisation de composés selon les revendications 1 à 7 pour la préparation de médicaments pour le traitement de l'hypertension, de l'hypertension pulmonaire, des insuffisances rénales aiguës et chroniques, des insuffisances cardiaques chroniques, de l'ischémie cérébrale, de la resténose après angioplastie, du cancer de la prostate.

9. Utilisation d'une combinaison d'un composé selon les revendications 1 à 7, avec un inhibiteur du système rénine-angiotensine.
